# EUROPÄISCHE PATENTSCHRIFT

(11) **EP 1 036 202 B1**
(45) Veröffentlichungstag und Bekanntmachung des Hinweises auf die Patenterteilung: **02.05.2002**
(21) Anmeldenummer: 98966608.6
(22) Anmeldetag: 04.12.1998
(51) Int. Cl.: C12Q 1/68

(54) **VERFAHREN ZUR IDENTIFIKATION VON NUCLEINSÄUREN DURCH MATRIX-ASSISTIERTE LASER DESORPTIONS/IONISATIONS MASSENSPEKTROMETRIE**
METHOD FOR IDENTIFYING NUCLEIC ACIDS BY MEANS OF MATRIX-ASSISTED LASER DESORPTION/IONISATION MASS SPECTROMETRY
PROCEDE D'IDENTIFICATION D'ACIDES NUCLEIQUES PAR SPECTROMETRIE DE MASSE PAR IONISATION/DESORPTION LASER ASSISTEE PAR MATRICE

(30) Priorität: 05.12.1997 EP 97121471
(43) Veröffentlichungstag der Anmeldung: 20.09.2000
(73) Patentinhaber: MAX-PLANCK-GESELLSCHAFT ZUR FÖRDERUNG DER WISSENSCHAFTEN E.V., 14195 Berlin (DE)
(72) Erfinder: GUT, Ivo, Glynne, 75014 - Paris (FR); BERLIN, Kurt, D-14532 Stahnsdorf (DE); LEHRACH, Hans, D-14195 Berlin (DE)
(74) Vertreter: VOSSIUS & PARTNER
(86) Internationale Anmeldenummer: EP9807911
(87) Internationale Veröffentlichungsnummer: WO9929898

(56) Entgegenhaltungen:
- EP-A- 0 524 808
- WO-A-95/04160
- WO-A-96/27681
- WO-A-98/31830
- LITTLE D P ET AL: "MALDI ON A CHIP: ANALYSIS OF ARRAYS OF LOW-FEMTOMOLE TO SUBFEMTOMOLE QUANTITIES OF SYNTHETIC OLIGONUCLEOTIDES AND DNA DIAGNOSTIC PRODUCTS DISPENSED BY A PIEZOELECTRIC PIPET" ANALYTICAL CHEMISTRY, Bd. 69, Nr. 22, 15. November 1997 (1997-11-15), Seiten 4540-4546, XP000727173 ISSN: 0003-2700
- GRIFFIN ET AL.: "GENETIC ANALYSIS BY PEPTIDE NUCLEIC ACID AFFINITY MALDI-TOF MASS SPECTROMETRY" NATURE BIOTECHNOLOGY, Bd. 15, Dezember 1997 (1997-12), Seiten 1368-1372, XP002113517
- ROSS ET AL.: "DISCRIMINATION OF SINGLE-NUCLEOTIDE POLYMORPHISMS IN HUMAN DNA USING PEPTIDE NUCLEIC ACID PROBES DETECTED BY MALDI-TOF MASS SPECTROMETRY" ANALYTICAL CHEMISTRY, Bd. 69, Nr. 20, Oktober 1997 (1997-10), Seiten 4197-4202, XP002113518
- ROSS P L & BELGRADER P: "ANALYSIS OF SHORT TANDEM REPEAT POLYMORPHISMS IN HUMAN DNA BY MATRIX-ASSISTED LASER DESORPTION/IONIZATION MASS SPECTROMETRY" ANALYTICAL CHEMISTRY, Bd. 69, 1. Oktober 1997 (1997-10-01), Seiten 3966-3972, XP002094185 ISSN: 0003-2700
- GUT ET AL.: "ANALYSIS OF DNA BY "CHARGE TAGGING" AND MATRIX-ASSISTED LASER DESORPTION/IONIZATION MASS SPECTROMETRY " RAPID COMMUNICATIONS IN MASS SPECTROMETRY, Bd. 11, 1997, Seiten 43-50, XP002113519
- KÖSTER ET AL.: "A STRATEGY FOR RAPID AND EFFICIENT DNA SEQUENCING BY MASS SPECTROMETRY" NAT.BIOTECH., Bd. 14, 1996, Seiten 1123-1128, XP002113520
- ARLINGHAUS H F ET AL: "MULTIPLEXED DNA SEQUENCING AND DIAGNOSTICS BY HYBRIDIZATION WITH ENRICHED STABLE ISOTOPE LABELS" ANALYTICAL CHEMISTRY, Bd. 69, Nr. 8, 15. April 1997 (1997-04-15), Seiten 1510-1517, XP000690164 ISSN: 0003-2700

## Beschreibung

Die vorliegende Erfindung betrifft ein Verfahren zum Nachweis einer Nucleotidsequenz in einem Nucleinsäuremolekül vermittels vorbestimmter Sonden unterschiedlicher Masse durch Matrix-assistierte Laser Desorptions/lonisations Massenspektrometrie. Vorteilhafterweise erlaubt das erfindungsgemäße Verfahren die gleichzeitige Charakterisierung einer Vielzahl unbekannter Nucleinsäuremoleküle durch einen Satz verschiedener Sonden. Weiterhin betrifft diese Erfindung einen Kit, der die Sonden und/oder einen Probenträger, gegebenenfalls mit daran gebundenen Nucleinsäuremolekülen, enthält.

Die genaue Charakterisierung von Nucleinsäuren ist sehr aufwendig und teuer. Unbekannte DNA kann charakterisiert werden, indem sie sequenziert wird. Dies ist die präziseste Art, DNA zu analysieren. Jedoch ist die Sequenzierung von DNA sehr aufwendig und nur dann notwendig, wenn die gesamte Sequenz von Interesse ist. Nur sehr kurze DNA-Ausschnitte (<1000 Nucleobasen) können in einem Arbeitsgang sequenziert werden. Wenn DNA Fragmente, die größer als diese 1000 Nucleobasen lang sind, in größerem Umfang analysiert werden sollen, ist es notwendig die DNA zu unterteilen, was die Methode verteuert.

Viele Aussagen lassen sich jedoch bereits mit geringerer Auflösung machen. Die derzeit beschriebenen Verfahren leiden jedoch darunter, daß unter Umständen Radioaktivität verwendet werden muß, und nur eine einzige Sonde in einer Analyse verwendet werden kann. Ein solches Verfahren aus dem Stand der Technik beinhaltet zum Beispiel, partielle Information über einen Array von verschiedenen Ziel-DNAs zu suchen. Ein Array mit vielen tausend Ziel-DNAs kann auf einer Festphase immobilisiert und anschließend können alle Ziel-DNAs gemeinsam auf das Vorhandensein einer Sequenz mittels einer Sonde (Nucleinsäure mit komplementärer Sequenz) untersucht werden.^{1,2} Eine Übereinstimmung in der Ziel-DNA mit der Sonde kann durch eine Hybridisation der beiden Nucleinsäuren nachgewiesen werden. Sonden können beliebige Nucleinsäuresequenzen unterschiedlicher Länge sein. Es existieren verschiedene Verfahren für die Auswahl von optimalen Bibliotheken von Sondensequenzen, welche minimal miteinander überlappen.^{3,4} Sondensequenzen können auch gezielt zusammengestellt werden, um bestimmte Ziel-DNA Sequenzen aufzufinden. Oligofingerprinting ist ein Ansatz, bei welchem diese Technologie zum Einsatz kommt. Eine Bibliothek von Ziel-DNAs wird mit kurzen Nucleinsäuresonden abgetastet. Meist sind hier die Sonden nur 8-12 Basen lang. Es wird jeweils eine Sonde auf einmal an eine auf einer Nylonmembran immobilisierte Ziel-DNA-Bibliothek hybridisiert. Die Sonde ist radioaktiv markiert und die Hybridisierung wird anhand der Lokalisierung der Radioaktivität beurteilt. Für die Abtastung eines immobilisierten DNA-Arrays sind auch bereits fluoreszent markierte Sonden verwendet worden.⁵ Ein ähnliches Verfahren wird verwendet, um Sequenzierung von DNA zu multiplexen.^{6,7} Verschiedene Vektorsysteme werden zur Klonierung von Zielsequenzen eingesetzt. Jeweils ein Klon von jedem Klonierungsvektor wird gepoolt, die Sequenzierungsreaktion durchgeführt, die Fragmente auf einem Gel aufgetrennt und das Gel auf eine Nylonmembran geblottet. An die immobilisierte DNA werden anschließend die unterschiedlichen Sequenzen des Klonierungssystems hybridisiert, so daß man die zum jeweiligen Klonierungssystem gehörende Sequenz erhält. Hier kann die Abtastung des Klonierungssystems auch mittels einer massenspektrometrisch detektierbaren Sonde erfolgen.⁸

Als Sonden kommen jegliche Moleküle in Frage, die sequenzspezifisch mit einer Ziel-DNA wechselwirken können. Am gängigsten sind Oligodeoxyribonucieotide. Für diesen Zweck bietet sich jedoch jede Modifikation von Nucleinsäuren an, z.B. Peptide Nucleic Acids (PNA),^{9,10} Phosphorothioatoligonucleotide oder Methylphosphonatoligonucleotide. Die Spezifität einer Sonde ist sehr wesentlich. Phosphorothioatoligonucleotide sind nicht besonders bevorzugt, da ihre Struktur durch die Schwefelatome verändert wird, was einen negativen Einfluß auf die Hybridisierungseigenschaften hat. Dies kann daher stammen, daß Phosphorothioatoligonucleotide normalerweise nicht diastereomerenrein synthetisiert werden. Bei Methylphosphonatoligonucleotiden bestand in der Vergangenheit ein ähnliches Reinheitsproblem, jedoch werden diese Oligonucleotide vermehrt diastereomerenrein synthetisiert. Ein wesentlicher Unterschied zwischen Methylphosphonatoligonucleotiden und Phosphorothioatoligonucleotiden ist das bei ersteren ungeladene Rückgrat, welches zu einer verminderten Abhängigkeit der Hybridisation von Puffersalzen und insgesamt durch die geringere Abstoßung zu höherer Affinität führt. Peptide Nucleic Acids haben ebenfalls ein ungeladenes Rückgrat, welches gleichzeitig chemisch sehr stark von der gängigen Zucker-Phosphat Struktur des Rückgrats in Nucleinsäuren abweicht. Das Rückgrat einer PNA weist eine Amidsequenz anstelle des Zucker-Phosphat Rückgrats gewöhnlicher DNA auf. PNA hybridisiert sehr gut mit DNA komplementärer Sequenz. Die Schmelztemperatur eines PNA/DNA-Hybrids ist höher als die des entsprechenden DNA/DNA-Hybrids und wiederum ist die Abhängigkeit der Hybridisierung von Puffersalzen relativ gering.

Matrix-assistierte Laser Desorptions/lonisations Massenspektrometrie (MALDI) ist eine neue, sehr leistungsfähige Methode für die Analyse von Biomolekülen.¹¹ Ein Analytmolekül wird in eine lichtabsorbierende Matrix eingebettet. Durch einen kurzen Laserpuls wird die Matrix verdampft und der Analyt so unfragmentiert in die Gasphase befördert. Durch Stöße mit Matrixmolekülen wird die Ionisation des Analyts erreicht. Eine angelegte Spannung beschleunigt die lonen in ein feldfreies Flugrohr. Auf Grund ihrer verschiedenen Massen werden Ionen unterschiedlich stark beschleunigt. Kleinere lonen erreichen den Detektor früher als größere. Die Flugzeit wird in die Masse der lonen umgerechnet. Technische Neuerungen der Hardware haben die Methode signifikant verbessert. Erwähnenswert ist delayed extraction (DE).¹² Für DE wird die Beschleunigungsspannung mit einer Verzögerung zum Laserpuls eingeschaltet und dadurch eine verbesserte Auflösung der Signale erreicht, weil die Zahl der Stöße verringert wird. MALDI eignet sich ausgezeichnet zur Analyse von Peptiden und Proteinen. Die Analyse von Nucleinsäuren ist schwieriger.¹³ Für Nucleinsäuren ist die Empfindlichkeit etwa 100mal. schlechter als für Peptide und nimmt mit zunehmender Fragmentgröße überproportional ab. Der Grund dafür liegt darin, daß für die Ionisation von Peptiden und Proteinen lediglich ein einziges Proton eingefangen werden muß. Für Nucleinsäuren, die ein vielfach negativ geladenes Rückgrat haben, ist der lonisationsprozeß durch die Matrix wesentlich ineffizienter. Für MALDI spielt die Wahl der Matrix eine eminent wichtige Rolle Für die Desorption von Peptiden sind einige sehr leistungsfähige Matrices gefunden worden, die eine sehr feine Kristallisation ergeben. Für DNA sind zwar mittlerweile einige ansprechende Matrices gefunden worden, jedoch wurde dadurch der Empfindlichkeitsunterschied nicht verringert. Der Empfindlichkeitsunterschied kann verringert werden, indem die DNA chemisch so modifiziert wird, daß sie einem Peptid ähnlicher wird. Phosphorothioatnucleinsäuren, bei denen die gewöhnlichen Phosphate des Rückgrats durch Thiophosphate substituiert sind, lassen sich durch einfache Alkylierungschemie in eine ladungsneutrale DNA umwandeln.¹⁴ Die Kopplung eines "charge tags" an diese modifizierte DNA resultiert in der Steigerung der Empfindlichkeit in den gleichen Bereich wie er für Peptide gefunden wird.^{15,16} Durch diese Modifikationen hat sich auch die Möglichkeit eröffnet, ähnliche Matrices wie sie für die Desorption von Peptiden verwendet werden zu benützen. Ein weiterer Vorteil von charge tagging ist die erhöhte Stabilität der Analyse gegen Verunreinigungen, die den Nachweis unmodifizierter Substrate stark erschweren. PNAs und Methylphosphonatoligonucleotide sind mit MALDI untersucht worden und lassen sich so analysieren.^{17,18,19}

T. J. Griffin et al. (1997) Nature Biotechnol. 15, 1368-1372, offenbart ein Verfahren zum Nachweis von polymorphische Stellen in DNA mit folgende Schritten: (i) Immobilisierung von biotinylierten doppel-strängiger DNA auf einem Streptavidin-beschichteten Träger, (ii) Denaturierung der immobilisierten DNAs, (iii) Hybridisierung von zwei PNA Sonden mit der Ziel-DNA, wobei eine Sonde die native Nucleotidsequenz des Alleles aufweist und die andere ein "one-base mismatch" aufweist (beide Sonden sind mit verschiedenen Massen-Tags versehen), (iv) Entfernung von nicht-hybridisiertes Material, (v) Analyse der immobilisierten PNA-DNA Hybride mittels MALDI-TOF, wobei Proben-spezifisch Massen-Spektren aufgenommen werden.

Kombinatorische Synthesen,²⁰ d.h. die Herstellung von Substanzbibliotheken ausgehend von einem Gemisch von Vorstufen, werden sowohl auf fester als auch in flüssiger Phase durchgeführt. Vor allem die kombinatorische Festphasensynthese hat sich frühzeitig etabliert, da in diesem Fall die Abtrennung von Nebenprodukten besonders einfach ist. Nur die an den Träger (Support) gebundenen Zielverbindungen werden in einem Waschschritt zurückbehalten und am Ende der Synthese durch das gezielte Spalten eines Linkers isoliert. Diese Technik erlaubt auf einfachem Wege die gleichzeitige Synthese einer Vielzahl verschiedener Verbindungen an einer Festphase und somit den Erhalt von chemisch "reinen" Substanzbibliotheken.^{21,22,23} Daher sind die Verbindungsklassen, die auch in nicht kombinatorischen, konventionellen Synthesen auf einer Festphase synthetisiert werden, der kombinatorischen Chemie besonders leicht zugänglich und werden demzufolge auch breit verwendet. Dies trifft vor allem auf Peptid-, Nucleinsäure- und PNA-Bibliotheken zu.

Die Synthese von Peptiden erfolgt durch Binden der ersten N-geschützten Aminosäure (z.B. Boc) an den Support, nachfolgende Entschützung und Reaktion der zweiten Aminosäure mit der freigewordenen NH₂-Gruppe der ersten. Nicht reagierte Aminofunktionen werden in einem weiteren "Capping" Schritt einer Weiterreaktion im nächsten Synthesezyklus entzogen. Die Schutzgruppe an der Aminofunktion der zweiten Aminosäure wird entfernt und der nächste Baustein kann gekoppelt werden. Zur Synthese von Peptidbibliotheken wird ein Gemisch von Aminosäuren in einem oder mehreren Schritten verwendet. Die Synthese von PNA und PNA-Bibliotheken erfolgt sinngemäß.

Nucleinsäure-Bibliotheken werden meist durch Festphasensynthese mit Gemischen verschiedener Phosphoramidit-Nucleoside erhalten. Dies kann auf kommerziell erhältlichen DNA-Synthesizern ohne Veränderungen in den Syntheseprotokollen durchgeführt werden. Darüber hinaus sind verschiedene Arbeiten zur kombinatorischen Synthese von PNA Bibliotheken publiziert worden.^{24,25} Diese Arbeiten behandeln den Aufbau von kombinatorischen Sequenzen, d.h. die Synthese von PNAs in denen einzelne, spezifische Basen in der Sequenz durch degenerierte Basen ersetzt werden und dadurch zufällige Sequenzvarianz erreicht wird. Auch die Verwendung massenspektrometrischer Methoden für die Analyse kombinatorischer Bibliotheken ist mehrfach beschrieben worden.^{26,27,28,29}

Es existieren verschiedene Verfahren, um DNA zu immobilisieren. Das bekannteste Verfahren ist die Festbindung einer DNA, welche mit Biotin funktionalisiert ist, an eine Streptavidin-beschichtete Oberfläche.³⁰ Die Bindungsstärke dieses Systems entspricht einer kovalenten chemischen Bindung, ohne eine zu sein. Um eine Ziel-DNA kovalent an eine chemisch vorbereitete Oberfläche binden zu können, bedarf es einer entsprechenden Funktionalität der Ziel-DNA. DNA selbst besitzt keine Funktionalisierung, die dazu geeignet ist. Es gibt verschiedene Varianten, in eine Ziel-DNA eine geeignete Funktionalisierung einzuführen: Zwei leicht zu handhabende Funktionalisierungen sind primäre, aliphatische Amine und Thiole. Solche Amine werden quantitativ mit N-Hydroxy-succinimidestern umgesetzt. Thiole reagieren unter geeigneten Bedingungen quantitativ mit Alkyliodiden. Eine Schwierigkeit besteht im Einführen einer solchen Funktionalisierung in eine DNA. Die einfachste Variante ist die Einführung durch einen Primer einer PCR. Gezeigte Varianten benützen 5'-modifizierte Primer (NH₂ und SH) und einen bifunktionalen Linker.^{31,32,33}

Bei der Immobilisierung auf einer Oberfläche ist vor allem ihre Beschaffenheit von wesentlicher Bedeutung. Bis jetzt beschriebene Systeme sind hauptsächlich aus Silizium oder Metall (magnetic beads). Eine weitere Methode zur Bindung einer Ziel-DNA basiert darauf, eine kurze Erkennungssequenz (z.B. 20 Basen) in der Ziel-DNA zur Hybridisierung an ein oberflächenimmobilisiertes Oligonucleotid zu verwenden.³⁴ Es sind auch enzymatische Varianten zur Einführung von chemisch aktivierten Positionen in eine Ziel-DNA beschrieben worden.³⁵ Hier wird an einer Ziel-DNA enzymatisch eine 5'-NH₂-Funktionalisierung durchgeführt.

Wie vorstehend beschrieben, sind im Stand der Technik eine Reihe von Verfahren bekannt, die vor allem auf eine genaue Analyse von Nucleinsäuren ausgerichtet sind. Diese Verfahren sind in der Regel entweder sehr arbeitsaufwendig und/oder kostenintensiv.

Das der vorliegenden Erfindung zugrundeliegende technische Problem war somit, ein schnelles und kosteneffizientes Verfahren zur Identifikation von Ziel-Nucieinsäuren bereitzustellen.

Dieses technische Problem wird durch die Bereitstellung der in den Patentansprüchen gekennzeichneten Ausführungsformen gelöst.

Somit betrifft die vorliegende Erfindung ein Verfahren zum Nachweis einer Nucleotidsequenz in einem Nucleinsäuremolekül, das die folgenden Schritte umfaßt:
(a) Hybridisierung von Nucleinsäuremotekülen mit einem Satz von Sonden unterschiedlicher Nucleobasensequenzen, wobei jede Sonde eine von allen anderen Sonden verschiedene Masse aufweist;
(b) Abtrennung der nicht hybridisierten Sonden;
(c) Kontaktierung der hybridisierten Sonden mit einer Matrix, welche die Desorption der Sonden durch einen Laserstrahl unterstützt;
(d) Analyse der hybridisierten und von der Matrix umgebenen Sonden auf einem aus elektrisch leitfähigem Material bestehenden Probenträger in einem Massenspektrometer; und
(e) Bestimmung der Nucleinsäuremoleküle, welche die Sequenz aufweisen, wobei die Positionen der Sonden auf dem Probenträger eine Zuordnung zu dem damit hybridisierenden Nucleinsäuremolekül erlaubt.

Das erfindungsgemäße Verfahren kombiniert in vorteilhafter Weise Verfahren, Arrays von Ziel-Nucleinsäuren zu analysieren (Oligofingerprinting), sowie die massenspektrometrische Analyse von Nucleinsäuren und modifizierten Nucleinsäuren. Dabei wird eine Vielzahl von unterschiedlichen Sonden eingesetzt, die den Nachweis von einer oder mehreren Nucleotidsequenz(en) in einem Nucleinsäuremolekül erlauben. Eine Kombination dieser beiden Methoden konnte bisher nicht durchgeführt werden, weil die Massenunterscheidbarkeit der Sonden keine eindeutigen Rückschlüsse auf die Sequenz zuließ und die Empfindlichkeit der massenspektrometrischen Analyse von Nucleinsäuren nicht an die Probenmengen eines Oligofingerprinting-Experimentes angepaßt war. Voraussetzung dafür, daß eine bestimmte Sequenz nachgewiesen werden kann, ist, daß im Verfahren Sonden eingesetzt werden, die eine Hybridisierung mit der Zielsequenz erlauben. Die Bestimmung der Nucleinsäuremoleküle erfolgt dabei über den Nachweis der Hybridisierung mit einer der Sonden.

Dem Fachmann ist klar, daß eine gesuchte Nucleotidsequenz mit Hybridisierungsverfahren nicht immer absolut ermittelt werden kann, da auch unter stringenten Hybridisierungsbedingungen trotz sogenannter "Mismatches" unter Umständen eine Hybridisierung einer Sonde stattfinden kann (z.B. ab einer bestimmten Mindestlänge einer Sonde oder bei (einer) Positionierung(en) des/der Mismatches, die bei der Hybridisierung tolerierbar ist/sind). Bei einer vorgegebenen Nucleotidsequenz einer Sonde kann eine Komplementärsequenz im Nudeinsäuremolekül somit in einem Teil der Ausführungsformen nur mit einer gewissen Näherung ermittelt werden, da neben exakten Komplementärsequenzen unter Umständen auch solche ermittelt werden können, die in ihrer Sequenz nicht exakt komplementär sind. Insofern umfaßt die Nucleotidsequenz auch homologe Nucleotidsequenzen, die vorzugsweise einen Homologiegrad von mehr als 90 %, besonders bevorzugt von mehr als 95 % aufweisen. Alle diese vorstehenden Ausführungsformen sind von der vorliegenden Erfindung umfaßt.

Je nach Wahl der Hybridisierungsbedingungen kann das erfindungsgemäße Verfahren verwendet werden, um entweder spezifische Nucleotidsequenzen oder Gruppen von Nucleotidsequenzen nachzuweisen, die sich durch eine ähnliche Sequenz auszeichnen. Werden z.B. stringente Hybridisierungsbedingungen gewählt, so sind die verwendeten Sonden nur in der Lage, an die Nucleotidsequenzen zu hybridisieren, die exakt komplementär zu ihren Nudeobasensequenzen sind. Werden hingegen nicht stringente Hybridisierungsbedingungen gewählt, so kann man mit den eingesetzten Sonden alle Nucleotidsequenzen nachweisen, die von den Nudeobasensequenzen der Sonden derart abweichen, daß sie unter den gewählten Bedingungen eine Hybridisierung noch erlauben. Auf diese Art und Weise kann das erfindungsgemäße Verfahren also auch verwendet werden, um Homologe, Varianten oder Allele einer bestimmten Sequenz nachzuweisen. Dem Fachmann ist bekannt, was unter stringenten bzw. nicht stringenten Hybridisierungsbedingungen zu verstehen ist; vgl. z. B. Sambrook et al., "Molecular Cloning, A Laboratory Manual" CSH Press, Cold Spring Harbor, 2nd ed. 1989, Hames und Higgins (Hrsg.) "Nucleic Acid Hybridization, A Practical Approach", IRL Press, Oxford 1985. Stringente Hybridisierungsbedingungen sind, zum Beispiel, Hybridisierung in 6 x SSC, 5 x Denhardt's Reagens, 0,5 % SDS und 100 µg/ml denaturierter DNA bei 65 °C und Waschen in 0,1 x SSC, 0,1 % SDS bei 65 °C. Nicht-stringente Hybridisierungsbedingungen unterscheiden sich von den obengenannten Bedinungen insofern, als beispielsweise die Hybridisierung und/oder das Waschen bei einer niedrigeren Temperatur durchgeführt werden, z.B. bei 50 °C oder 55 °C und/oder die Menge von SSC auf z.B. 1 x oder 2 x SSC angehoben wird.

Das erfindungsgemäße Verfahren erlaubt auch den Nachweis mehrerer unterschiedlicher Sequenzen in einer Ziel-DNA, wobei die unterschiedlichen Sequenzen komplementär zu verschiedenen Sonden sind. Günstigstenfalls, z. B. bei Verwendung von Sonden mit überlappenden Sequenzen, läßt sich so die gesamte Nucleotidsequenz einer Ziel-Nucleinsäure nachweisen oder aufklären.

Mit dem erfindungsgemäßen Verfahren läßt sich zunächst bestimmen, ob auf dem Probenträger eine Probe aufgetragen war, die eine mit einer Sonde unter den gewählten Bedingungen hybridisierbare Sequenz aufweist. Sofern dies der Fall ist, kann die Proben-Nucleinsäure weiter analysiert und charakterisiert werden. Da für das erfindungsgemäße Verfahren üblicherweise nur ein Bruchteil einer Probe für die erfindungsgemäße Analyse eingesetzt werden muß, kann die nicht eingesetzte Nucleinsäure mit Standardverfahren, z. B. Sequenzierungsverfahren, weiter untersucht werden.

Das erfindungsgemäße Verfahren kann auch, parallel oder sukzessive, mehrmals durchgeführt werden, wobei die Hybridisierungsbedingungen variiert werden. So kann man beispielsweise in einem Ziel-DNA-Array zunächst feststellen, wie viele und welche Ziel-DNAs einen bestimmten Homologiegrad aufweisen, bevor nach spezifischen Sequenzen gesucht wird.

Die Zuordnung der an einer bestimmten Position auf dem Probenträger hybridisierten Sonde zur immobilisierten Probe erfolgt vorzugsweise mittels eines Datenverarbeitungssystems, das das jeweilige aufgenommene Spektrum auf einer Position des Probenträgers einer auf derselben Position angebrachten Ziel-DNA zuordnet. Vorzugsweise sind die Ziel-Nucleinsäuren auf der Oberfläche bzw. auf dem Proteinträger in einer bestimmten Ordnung angeordnet.

Wie vorstehend erwähnt, besteht der Probenträger aus einem elektrisch leitfähigem Material. Dies schließt auch die Oberfläche des Probenträgers ein, auf dem die hybridisierten und von der Matrix umgebenen Sonden aufsitzen.
Die Oberfläche kann sich dabei stofflich vom Probenträger unterscheiden.

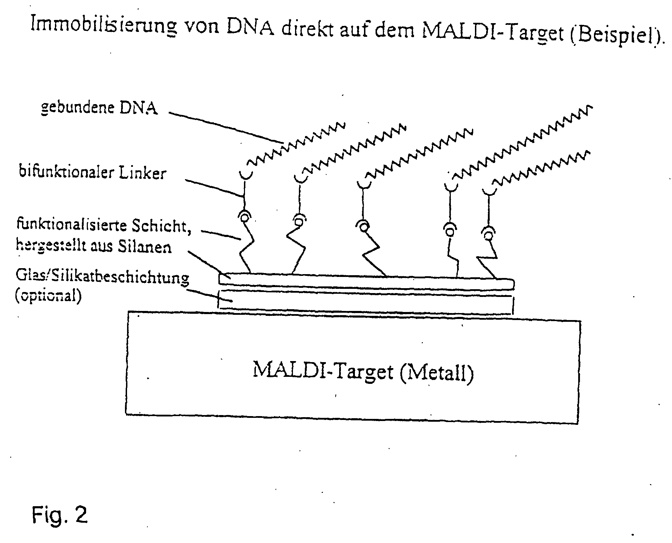

Die Oberfläche, an der die Sonden für die Massenspektrometrie direkt oder indirekt immobilisiert sein müssen, muß so beschaffen sein, daß sie als Probenträger für ein Massenspektrometer fungieren kann (Figur 2). Dies bedeutet, daß sie aus einem elektrisch leitfähigen Material bestehen muß, da, um eine stabile Beschleunigung der ionisierten Sondenmoleküle zu erreichen, eine definierte Spannung angelegt werden muß. Eine nichtleitende Oberfläche würde zu einer elektrostatischen Aufladung führen, wodurch wegen der Spannungsabweichung eine Massenverschiebung beobachtet und eine Massenzuordnung unmöglich gemacht würde.

Bevorzugte Ausführungsformen der vorliegenden Erfindung sind nachstehend beschrieben und können auch den Beispielen entnommen werden.

In einer bevorzugten Ausführungsform des erfindungsgemäßen Verfahrens werden die Nudeinsäuremoleküle vor oder nach Schritt (a) auf die Oberfläche eines Trägers übertragen. Als Träger eignen sich dazu z.B. "magnetic beads", Kunststoffbeads oder Glasbeads, die beispielsweise mit Streptavidin oder mit Aminogruppen, SH-Gruppen oder Epoxygruppen funktionalisiert sind.

In einer besonders bevorzugten Ausführungsform handelt es sich bei der Oberfläche des Trägers um die Oberfläche des aus elektrisch leitfähigem Material bestehenden Probenträgers. Diese Ausführungsform erlaubt eine relativ unkomplizierte Durchführung des Verfahrens, da die Nucleinsäuremoleküle nach erfolgter Hybridisierung nicht mehr auf den Probenträger transferiert werden müssen.

In einer weiteren besonders bevorzugten Ausführungsform des erfindungsgemäßen Verfahrens wird vor Schritt (c) der Träger mit den an seiner Oberfläche aufgebrachten Nucleinsäuremolekülen, die die hybridisierten Sonden tragen, auf den aus elektrisch leitfähigem Material bestehenden Probenträger aufgebracht. Die Anheftung kann z.B. durch die Matrix selbst oder durch Zugabe von Nitrozellulose zur Matrix bewirkt werden.

In einer ebenfalls besonders bevorzugten Ausführungsform werden die hybridisierten Sonden vor dem, nach dem oder durch den Kontakt mit der Matrix in Schritt (c) von den immobilisierten Nucleinsäuremolekülen abgelöst und auf den aus leitfähigem Material bestehenden Probenträger aufgebracht. In dieser Ausführungsform werden die Komplexe aus hybridisierten Nucleinsäuremolekülen und Sonden denaturiert, und nur die Sonden auf den Probenträger aufgebracht. Eine Denaturierung kann durch bekannte Methoden wie z.B. alkalische oder Hitze-Denaturierung bewirkt werden oder auch durch die saure Matrixlösung selbst.

In einer anderen bevorzugten Ausführungsform besitzt der Probenträger entweder eine metallische, mit Glas beschichtete oder chemisch modifizierte Oberfläche, die ein Festbinden der Ziel-DNA erlaubt.

In einer weiteren bevorzugten Ausführungsform des erfindungsgemäßen Verfahrens erfolgt die Immobilisierung der Nucleinsäuremoleküle an der Probenträgeroberfläche über eine NH₂-, Epoxy- oder SH-Funktion, vermittels der Beschichtung der Probenträgeroberfläche mit einem Silikat oder Silan, über eine Protein-Substrat-, Protein-Protein- oder eine Protein-Nucleinsäure-Interaktion, oder über eine Wechselwirkung zweier hydrophober Komponenten.

Wird der Probenträger z.B. mit Gold beschichtet, kann eine Kopplung der Ziel-DNA durch während der molekularbiologischen Aufbereitung der Ziel-DNA eingeführte SH- oder NH₂-Funktionen erfolgen. Auch die umgekehrte Möglichkeit, entsprechend modifizierte DNA an funktionalisierte Goldpartikel zu binden, besteht. Die Firma Nanoprobes Inc., Stony Brook, NY, bietet beispielsweise mit Streptavidin oder mit Aminofunktionen verknüpfte Gold-Nanopartikel kommerziell an. Wie bereits erwähnt, besteht eine weitere Möglichkeit darin, die metallische Probenträgeroberfläche mit Glas zu beschichten. Durch eine Aminofunktionalisierung kann anschließend eine Kopplung der Ziel-DNA, welche über eine SH-Funktionalisierung an einen bifunktionalen Linker (z.B. SIAB, Pierce Chemical, Rockford, IL, USA) gebunden ist, an die Glasoberfläche erreicht werden. Eine weitere Variante ist die unmittelbare Beschichtung der Metalloberfläche mit Trimethoxy-3-aminopropylsilan. An die Aminofunktion kann anschließend eine Ziel-DNA, wie oben über einen bifunktionalen Linker, gekoppelt werden.

In einer besonders bevorzugten Ausführungsform ist dabei die Protein-Substrat-Interaktion eine Biotin-Streptavidin- oder eine Antikörper-Antigen-Bindung. MALDI-Probenträger sind üblicherweise aus Metall (z. B. Eisen), an das sich ohne weitere Modifikation weder Proteine noch DNA-Moleküle immobilisieren lassen. Eine Möglichkeit der Immobilisierung ist, die Eisenoberfläche mit Gold zu beschichten, da sich z. B. SH-Funktionen daran binden lassen. Für die Kopplung bieten sich dann bifunktionale Linker an, die eine SH-Funktion und eine weitere Funktion besitzen, die an die Funktionalisierung der Ziel-DNA angepaßt ist. Ist z. B. die Ziel-DNA biotinfunktionalisiert, sollte der Linker mit Streptavidin gekoppelt werden können. Ist die Ziel-DNA NH₂-funktionalisiert, dann kann der Linker mit einer N-hydroxysuccinimidylester-Funktion versehen sein.

In einer weiteren besonders bevorzugten Ausführungsform ist die Protein-Nucleinsäure-Interaktion eine Bindung der Nucleinsäure an Gene32, ein Protein, welches sequenzunspezifisch einzelsträngige DNA bindet.

In einer ebenfalls bevorzugten Ausführungsform des erfindungsgemäßen Verfahrens sind die verwendeten Sonden Nucleinsäuren, die mit einem Massen-Tag versehen sind. Gemäß dieser Ausführungsform können die Sonden auch mit mehreren Tags versehen sein, die an unterschiedlichen Positionen z. B. am 5' und 3' Ende lokalisiert sind. Durch Kombination der Anzahl und Lokalisierung von Massen-Tags, gegebenenfalls in Kombination mit Ladungs-Tags, läßt sich die Versalität und Empfindlichkeit des erfindungsgemäßen Verfahrens deutlich erhöhen.

In einer besonders bevorzugten Ausführungsform sind die Massen-Tags zugleich Ladungs-Tags, während in einer weiteren besonders bevorzugten Ausführungsform die Nucleinsäuren zusätzlich mit einem Ladungs-Tag ("Charge-Tag") versehen sind.

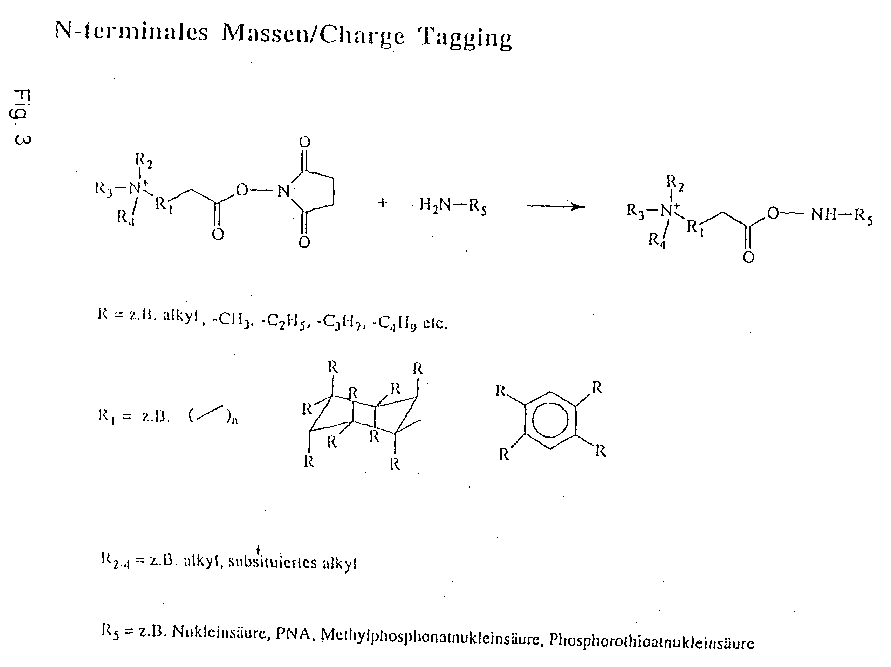

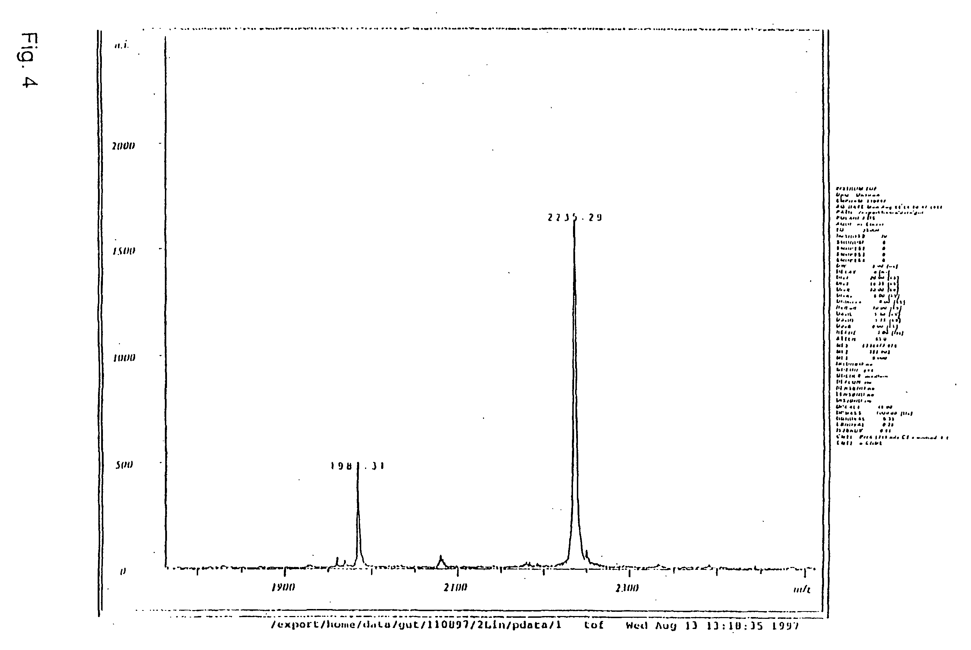

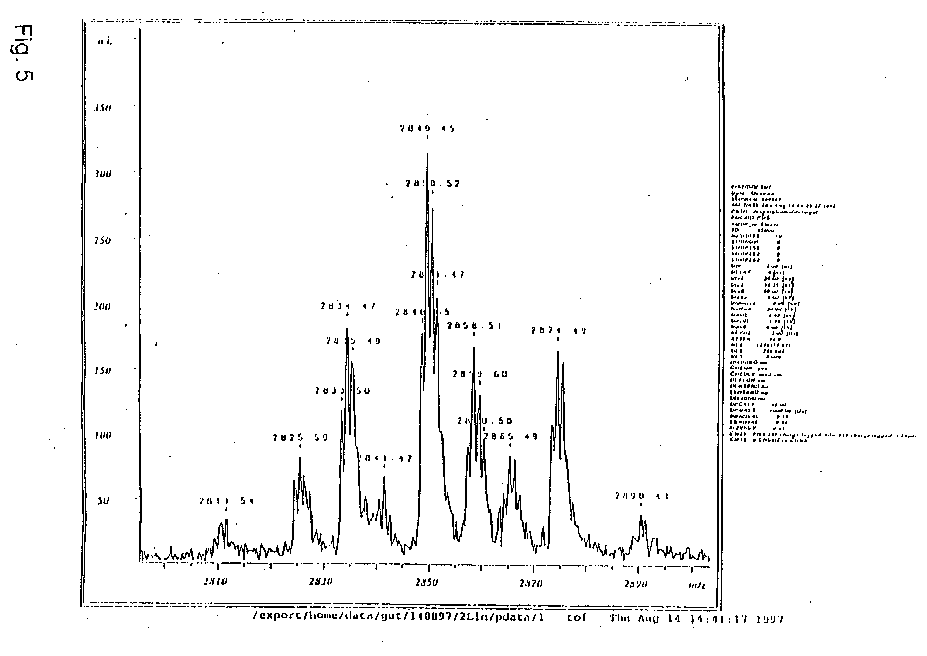

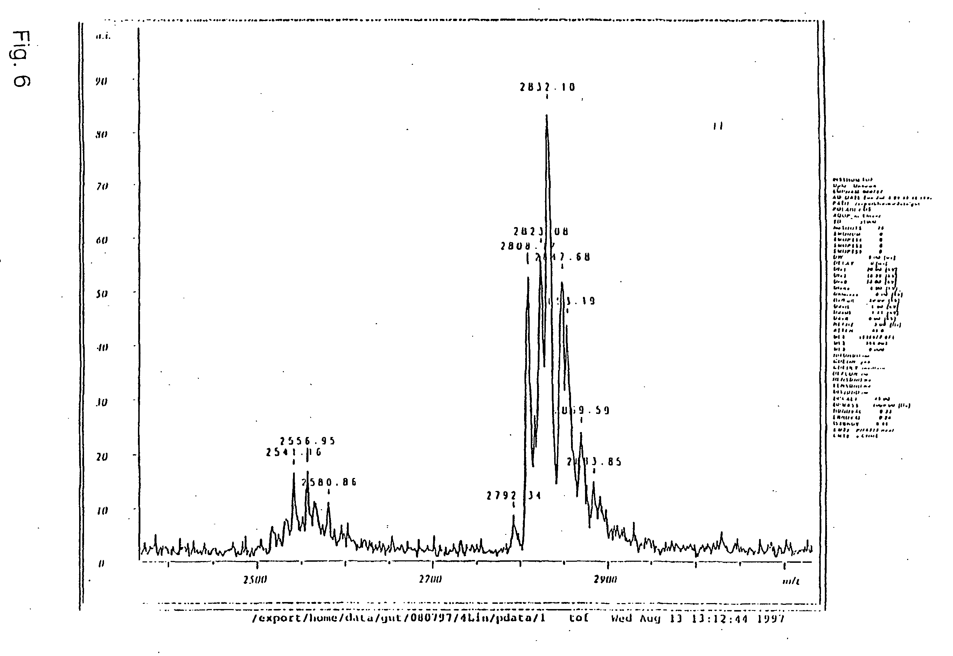

"Charge tagging" kann entsprechend der Methode von Gut et al. durchgeführt werden.^{15,16} Ein aminofunktionalisiertes Substrat (1 mM) wird in Trimethylamin/CO₂-Puffer (pH = 8.5, 200 mM) auf Eis bei 0 °C mit 1% ω-Trimethylammoniumhexansäure-N-hydroxy-succinimidylester (CT) versetzt. Nach 30 Minuten werden der flüchtige Puffer und das Lösungsmittel im Vakuum abgezogen. Das aminofunktionalisierte Substrat kann z.B. eine kombinatorisch hergestellte Bibliothek von massenverschiedenen Sonden sein. Durch die Variation der Länge und Funktionalisierung des CT lassen sich die Massen der Substratbibliothek um einen definierten Betrag verschieben (Fig. 3). Da in der kombinatorischen Synthese z.B. eine Sondenbibliothek von 64 massenverschiedenen Sonden (Fig. 4). die sich über einen Massenbereich von 200 Da erstrecken, hergestellt werden, erhöhen die Massen/charge tags jeweils in Einheiten von 200 Da die Masse. d.h. der erste kombinatorische Syntheseansatz wird mit dem kleinstmöglichen charge tag hergestellt, der zweite mit einem Massen/charge (Ladungs-) tag, das 200 Da schwerer ist, der dritte mit einem Massen/charge tag das weitere 200 Da schwerer ist usw. Theoretisch kann der Bereich beliebig ausgeweitet werden, solange das angewendete Massenspektrometer in der Lage ist, den Unterschied zwischen zwei massenbenachbarten Sonden aufzulösen und solange die Synthese präparativ praktikabel erscheint. Für Sonden mit 10 Nucleobasen bekommt man ein Grundmasse im Bereich von 2600-2800 Da. Mit derzeit käuflichen Massenspektrometern ist der benützbare Massenbereich mit ausreichender Massengenauigkeit unter 4000 Da Dadurch können sieben Ensembles von 64 Sonden verwendet werden (insgesamt 448 Sonden). Ergebnisse, die mit dieser Ausführungsform erhalten worden sind, sind in den Figuren 5 und 6 dargestellt.

Die Synthese von Peptiden auf einem automatisierten Synthesizer verläuft vom C-terminalen Ende zum N-terminalen Ende, die Synthese von Nucleinsäuren vom 3'- zum 5'-Ende. Es besteht die Möglichkeit an einem oder an beiden Enden eine primäre Aminofunktion anzubringen, um dann durch eine oder zwei Funktionalisierungen die Massenverschiebung zu erreichen. Alternativ kann die Massenverschiebung einer Bibliothek von kombinatorisch hergestellten Sonden auch erreicht werden, indem vorgängig zum Einbau der kombinatorischen Bausteine einige Bausteine von definierter Masse (z.B. Aminosäuren in eine kombinatorische Synthese von PNAs) angebracht werden. Die erste kombinatorische Synthese beginnt unmittelbar am Support. Für die zweite kombinatorische Synthese werden zuerst z.B. zwei Valine gekoppelt. Valin hat eine Masse von 99 Da. Durch zwei Valine erreicht man eine Massenverschiebung der zweiten kombinatorischen Synthese von der ersten um 198 Da. Bei der dritten kombinatorischen Synthese werden zuerst vier Valine gekoppelt, wodurch die Masse dieses Ensembles 396 Da höher ist usw. Ein allfällig nötiges charge tag kann immer noch nachträglich mit der oben beschriebenen Methode am N-terminalen Ende angebracht werden. Eine weitere Möglichkeit besteht darin, die charge tags zuerst an die Festphase zu koppeln und anschließend mit der kombinatorischen Synthese fortzufahren.

Eine weitere Variante besteht darin, gleichzeitig mehrere fixe Ladungen der gleichen Polarität anzubringen. Dadurch kann der Detektionsbereich vermindert werden. In einem Massenspektrometer wird ein Molekül mit zwei Ladungen bei der entsprechend halben Masse beobachtet. Dies kann einen Vorteil bringen, wenn die Auflösung des Massenspektrometers zu höheren Massen hin stark abnimmt. Oben ist beschrieben, wie man positive fixe Ladungen an einer Sonde festbringen kann. Negative fixe Ladungen können ähnliche Vorteile bringen. Positive und negative fixe Ladungen lassen sich mit ähnlichen Methoden herstellen.

Für die Lokalisierung, Anzahl und Kombinatorik der Ladungs-Tags gelten die im Zusammenhang mit den Massen-Tags gemachten Ausführungen entsprechend. So kann mittels charge tagging am 5'- oder 3'- Terminus der Bibliothek von Nucleinsäure-Sonden die Empfindlichkeit der Analyse verbessert werden. Dies gilt insbesondere, wenn durch die Einführung von Alkylphosphonatgruppen an den nicht randomisierten Positionen (vgl. unten) ansonsten Ladungsneutralität hergestellt wird. Durch das mit dem Charge Tagging zwangsläufig verbundene Massen Tagging wird zusätzlich die Analyse der Fragmente aus der Spaltung an den Phosphorothioatgruppen erleichtert. Phosphoroselenoate können ebenfalls eingesetzt werden, um eine bevorzugte Spaltung an bestimmten Positionen zu erhalten.

In einer weiteren bevorzugten Ausführungsform des erfindungsgemäßen Verfahrens sind die Sonden modifizierte Nucleinsäuremoleküle.

In einer besonders bevorzugten Ausführungsform handelt es sich bei diesen modifizierten Nucleinsäuremolekülen um PNAs, alkylierte Phosphorothioatnucleinsäuren oder Alkylphosphonatnucleinsäuren.

In einer weiteren bevorzugten Ausführungsform werden die Sonden des erfindungsgemäßen Verfahrens durch kombinatorische Festphasensynthese hergestellt. Das erfolgt über z.B. über die übliche Boc-Festphasensynthese, die auch zur Synthese von PNA-Reinsubstanzen kommerziell angewandt wird. Dabei werden in ausgewählten Positionen innerhalb einer sonst festgelegten Sequenz der Sonden-PNA alle vier, oder mehr als eine der Basen Adenin, Guanin, Cytosin oder Thymin, nebeneinander eingebaut. Dies erfolgt durch den Einsatz nicht nur eines, sondern mehrerer Synthesebausteine in einem Schritt in der Festphasensynthese. Durch Variationen in mehreren solchen Positionen entsteht eine PNA-Bibliothek. Im Anschluß an die PNA-Synthese kann ein charge tagging an der freien terminalen Aminofunktion durchgeführt werden. Dies verbessert den dynamischen Bereich der PNA-Analyse mittels MALDI.

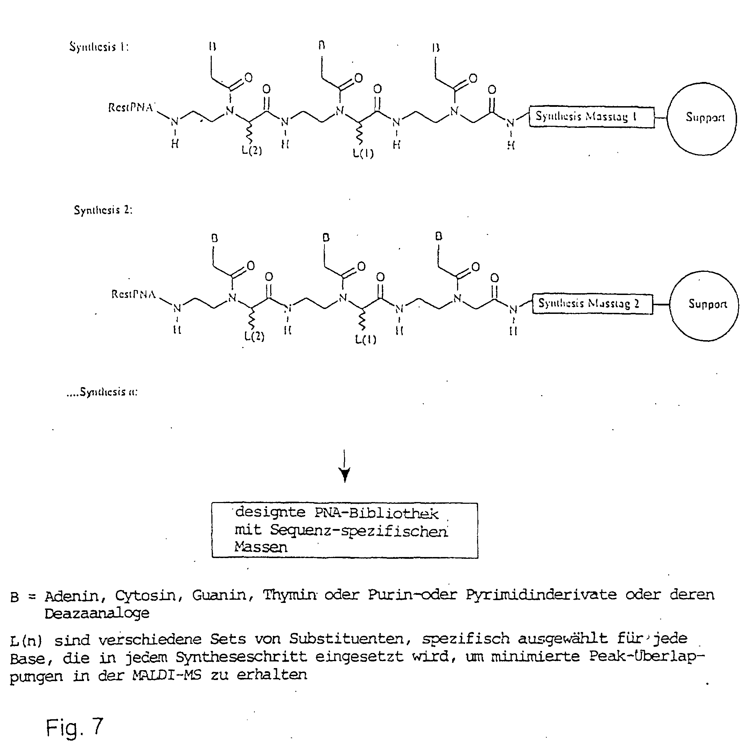

In einer besonders bevorzugten Ausführungsform werden deshalb bei der Festphasensynthese die unterschiedlichen Basenbausteine derart markiert, daß ihre Massen bzw. die Massen der aus ihnen synthetisierten Sonden im Massenspektrometer unterscheidbar sind. Diese Markierung wird so bewerkstelligt, daß zur Base korrespondierend am Rückrat eine unterschiedliche Massenmodifikation eingeführt wird (Figur 7). Auf diese Art und Weise erhalten die synthetisierten Sonden Massen, die spezifisch für ihre Sequenzen sind. Binden nun Sonden an eine auf dem MALDI-Target immobilisierte Ziel-DNA, so erlauben die im MALDI-Experiment zugänglichen Masseninformationen eindeutige Rückschlüsse auf die Sequenzen der jeweils hybridisierten PNA-Sonden.

In einer weiteren besonders bevorzugten Ausführungsform des erfindungsgemäßen Verfahrens werden die Basenbausteine mit einer Methyl-, Ethyl-, Propyl-, eine verzweigte oder unverzweigte Alkyl-, eine halogensubstituierte verzweigte oder unverzweigte Alkyl-, Alkoxyalkyl-, Alkylaryl-, Arylalkyl-, Alkoxyaryl- oder Aryloxyalkyl-Gruppe oder mit einer von deren deuterierten oder sonstigen isotopen Varianten markiert. Da bei Verwendung nicht massenmodifizierter Bausteine die Molekülmasse allenfalls Rückschlüsse auf die Basenzusammensetzung, nicht aber auf die Sequenz erlaubt, werden massengelabelte Bausteine (d.h. am Rückgrat substituierte PNA Monomere) verwendet, die für jede randomisierte Position innerhalb der PNA-Bibliothek charakteristisch sind. Da auf diese Weise der Synthesebaustein einer bestimmten Base in der Position x eine andere Masse hat als in der Position y, werden auch unterschiedliche Sequenzen bei gleicher Bruttozusammensetzung der Basen über die Molekülmasse unterscheidbar. Die jeweiligen Substituenten werden durch numerische Rechnung so ausgewählt, daß jeder denkbaren Sequenz aufgrund der beschriebenen Art der Bibliothekssynthese eine festgelegte Masse eineindeutig entspricht.

Die Synthese von Nucleinsäure-Sonden-Bibliotheken erfolgt am Synthesizer, indem an den zu randomisierenden Positionen Gemische verschiedener Nucleosidderivate (in der Regel Phosphoramidite) eingesetzt werden. Die sich daraus ergebenden Bibliotheken können ebenfalls als Sonden im oben beschriebenen Verfahren eingesetzt werden. Zur Unterscheidung der verschiedenen Sequenzen innerhalb der Bibliothek soll ein gezielter Bindungsbruch z.B. an festgelegten Phosphodiesterbindungen durchgeführt werden, die sich auf einer festgelegten Seite der randomisierten Positionen befinden.

In einer weiteren bevorzugten Ausführungsform des erfindungsgemäßen Verfahrens sind somit die Sonden mit mindestens einer Modifikation in einer definierten Position von randomisierten Nucleotiden entfernt versehen, die eine Spaltung der Sonde erlauben.

In einer besonders bevorzugten Ausführungsform handelt es sich bei dieser Modifikation um die Einführung einer Phosphorothioatgruppe und/oder einer RNA-Base und/oder einer Phosphotriesterbindung.

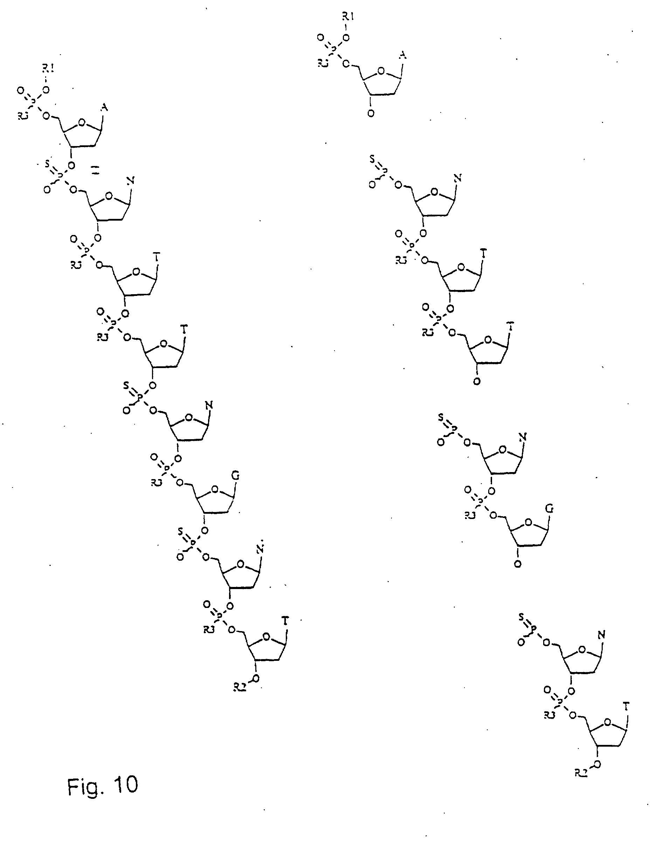

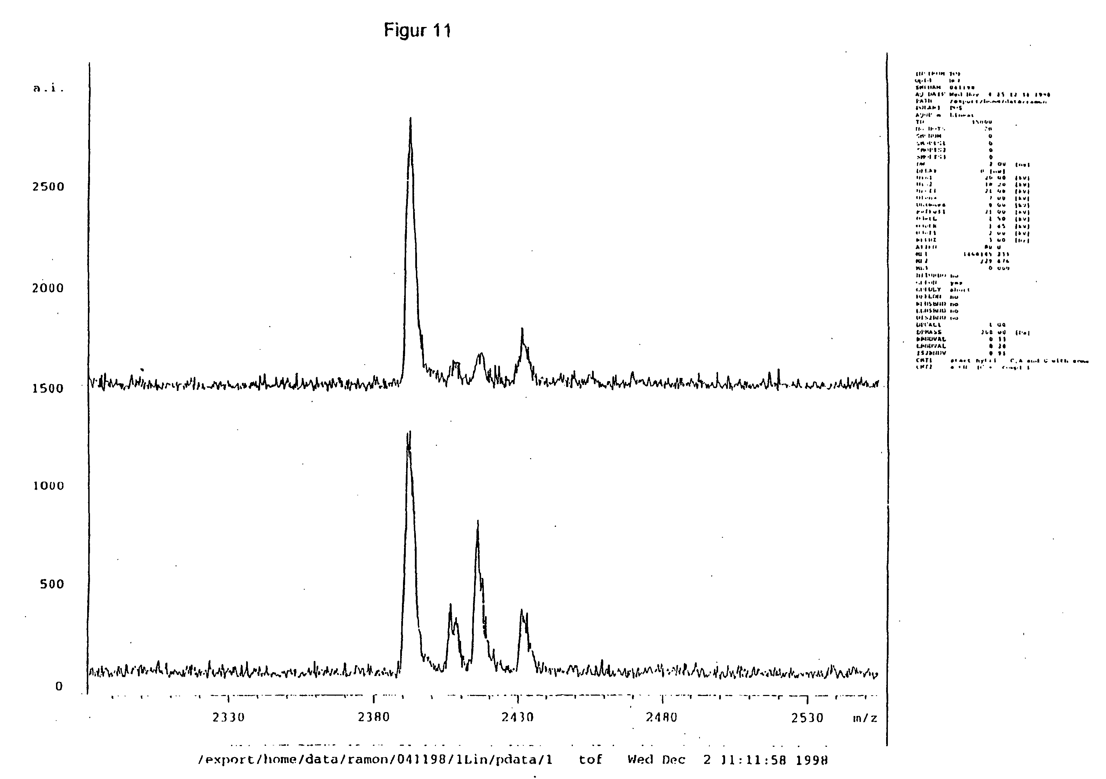

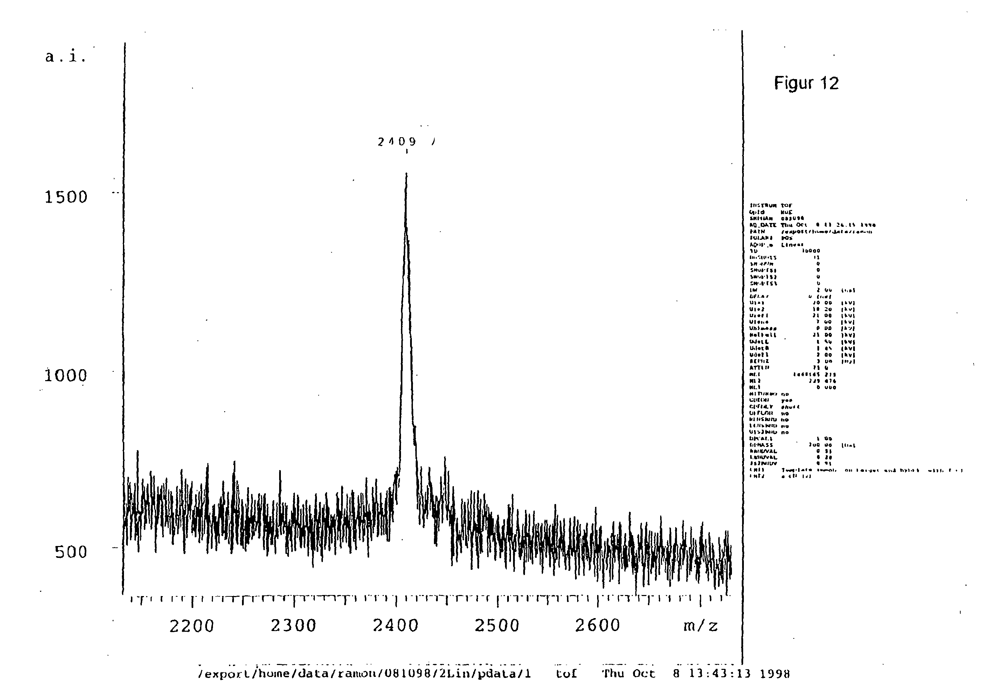

Enthält eine Sonde drei randomisierte Positionen, so sind mindestens zwei solcher Bindungsbrüche erforderlich (Figur 10). Es ergeben sich dann drei Fragmente, die jeweils eine randomisierte Position enthalten und damit aufgrund der sonst bekannten Zusammensetzung über ihre Masse unmittelbar auf die variable Base schließen lassen. Die Spaltung an den beschriebenen Bindungen kann unvollständig erfolgen. Dies erlaubt die Einbeziehung größerer Fragmente, um die Sequenzinformation abzusichern oder bei Mehrdeutigkeiten zu konkretisieren. Der spezifische Bindungsbruch an den randomisierten Positionen wird durchgeführt, indem bereits bei der Synthese der. Bibliothek dort Phosphorothioatgruppen eingeführt werden. Diese können mit Hydroxyalkyihalogeniden zuerst hydroxyalkyliert und dann unter basischen Bedingungen selektiv gespalten werden. Alternativ können Proben so aufgebaut werden, daß neben einer randomisierten Position Uracil eingebaut wird. Mittels Uracil-DNA Glycosylase und nachfolgender alkalischer Behandlung kann das Rückgrat dann an dieser Stelle gebrochen werden.

Die Matrix wird üblicherweise so gewählt, daß sie bei der gewählten Laserwellenlänge einen hohen Extinktionskoeffizienten sowie eine gute Unterstützung der Ladungsbildung aufweist. In einer weiteren bevorzugten Ausführungsform des erfindungsgemäßen Verfahrens besteht die Matrix, die die Desorption der Sonden durch den Laserstrahl unterstützt, aus einer Lösung aus α-Cyano-4-hydroxyzimtsäure in Aceton im Verhältnis 1:9 bis 9:1, vorzugsweise im Verhältnis 1:1, oder einem Gemisch aus α-Cyano-4-hydroxyzimtsäuremethylester und α-Cyano-4-methoxyzimtsäure oder Sinapinsäure oder deren Methylderivate im Verhältnis 1:9 bis 9:1, vorzugsweise im Verhältnis 1:1.

In einer anderen bevorzugten Ausführungsform des erfindungsgemäßen Verfahrens besteht die Matrix aus α-Cyano-4-hydroxyzimtsäure oder einem Gemisch aus α-Cyano-4-hydroxyzimtsäuremethylester und α-Cyano-4-methoxyzimtsäure oder α-Cyano-4-hydroxyzimtsäure oder Sinapinsäure oder deren Methylderivate im Verhältnis 1:99 bis 99:1, vorzugsweise im Verhältnis 1:1, die als Lösung in Aceton, Isopropanol, Acetonitril, Ethanol, Methanol oder Wasser oder in einem Gemisch aus zweien oder mehreren dieser Lösungsmittel auf den MALDI-Probenträger aufgebracht wird.

Das oben beschriebene Prinzip des Massentaggings für bestimmte Bausteine in festgelegten Positionen kann für verschiedene Teilbibliotheken angewendet werden, die dann wiederum zu einer größeren Bibliothek vereinigt werden können.

In einer weiteren bevorzugten Ausführungsform werden deshalb die Sonden als Teilbibliotheken hergestellt, die mit unterschiedlichen Massen- und/oder Ladungs-Tags versehen sind. Um von einer bestimmten analysierten Masse auf eine bestimmte Teilbibliothek rückschließen zu können, müssen auch diese Teilbibliotheken in ihrer Synthese massengelabelt werden. Dies erfolgt zweckmäßig über natürliche Aminosäuren, die sich leicht in einer Festphasensynthese an die PNA Bibliothek binden lassen. Durch das Massenlabeln verschiedener Teilbibliotheken läßt sich der Massenbereich, der für die Analyse der Gesamtbibliothek zur Verfügung steht, beträchtlich aufweiten.

Weiterhin betrifft die vorliegende Erfindung einen Kit, der die Sonden und/oder einen Probenträger, gegebenenfalls mit den gebundenen Nucleinsäuremolekülen, enthält. Der Proteinträger ist, wie vorstehend dargelegt, oberflächenvorbehandelt und erlaubt so die Bindung der Nucleinsäuren. Vorzugsweise erfolgt die Vorbehandlung auf chemischem Wege.

Die Figuren zeigen:

### Figur 1

### Schema des Fingerprintings mit massenspektrometrischer Abtastung.

1) Kombinatorisch hergestellte Bibliothek von massenunterscheidbaren Sonden. 2) Die Bibliothek wird an eine Ziel-DNA, die auf einem MALDI target immobilisiert ist, hybridisiert. Nur Sonden mit komplementären Sequenzen zu Sequenzen in der Ziel-DNA setzen sich an der Ziel-DNA fest. Nach intensivem Waschen wird eine MALDI-Matrix aufgebracht. 3) Mit einer Massenanalyse werden die hybridisierten Sonden identifiziert.

### Figur 2

Schematische Darstellung der Beschichtung eines MALDI-Targets und Immobilisierung eines Arrays von Ziel-DNAs. Der bifunktionale Linker wird bei nicht kovalenter Immobilisierung weggelassen.

### Figur 3

### N-terminales Massen/charge Tagging.

Am 5' einer Nucleinsäure oder einer modifizierten Version einer Nucleinsäure oder am N-terminalen Ende einer PNA kann ein charge tag eingeführt werden. Das charge tag trägt eine N-Hydroxy-succinimidester Funktion und eine quarternäre Ammoniumgruppe. Diese zwei Funktionalisierungen sind durch eine Gruppe R₁ getrennt. R₁ dient der Massenvariation der charge tags (die Kopplung der charge tags wird bei leicht basischem pH (8.5) in wäßriger Lösung auf Eis durchgeführt. Die Reaktion läuft in 30 Minuten vollständig ab).

### Figur 4

### 1:1 Mischung von unmodifizierter PNA und charge tag PNA.

Eine charge tag und unmodifizierte PNA sind in einem 1:1 Gemisch mit einer α-Cyano-4-hydroxyzimtsäuremethylester Matrix analysiert worden. Die unterschiedliche Empfindlichkeit ist offensichtlich. Die um eine Base verkürzte, gechargetagte Fehlersequenz (1983 Da) ergibt ein wesentlich stärkeres Signal als die unmodifizierte Sequenz (2074 Da).

### Figur 5

### Massenverteilung einer 10-mer PNA Bibliothek.

Zwei Positionen in der Sequenz sind mit degenerierten Basen (TCGA) synthetisiert. Die Sequenz ist NTTGTTTTCN. Dadurch ergeben sich 9 massenunterscheidbare PNAs. In der PNA Bibliothek ist CC = 2810 Da, CT = TC = 2825 Da, CA = AC = 2834 Da, TT = 2840 Da, TA = AT = 2849 Da nicht unterscheidbar von CG = GC = 2850 Da, AA = 2858 Da, TG = GT = 2865 Da, AG = GA = 2874 Da und GG = 2890 Da. Durchwegs wird isotope Auflösung beobachtet. Die ganze PNA Bibliothek ist in einer Eintopfreaktion mit einem charge tag versehen worden. Die MALDI Analyse wurde in einem 1:1 Matrixgemisch von α-Cyano-4-methoxyzimtsäure und α-Cyano-4-hydroxyzimtsäuremethylester durchgeführt.

### Figur 6

### Massen/Charge tagging.

Eine 10-mer PNA Bibliothek mit 2 degeneriert synthetisierten Positionen sind mit einem Massen/Charge tag gelabelt worden. Die Sequenz ist NTTGTTTTCN. Gezeigt ist ein Gemisch von Ausgangsmaterial und gelabelter PNA Bibliothek in einer α-Cyano-4-hydroxyzimtsäure Matrix. Der Vorteil des Anbringens eines Fixladungsträgers an die Bibliothek ist offensichtlich. Die Signale von der charge getagten Bibliothek sind bei gleicher Konzentration detektierbar, während die unmodifizierte PNAs nicht zu detektieren sind.
Die um eine Base verkürzte Fehlersequenz ist zu erkennen.

### Figur 7

Prinzip der Synthese von PNA-Bibliotheken unter Verwendung parallel durchgeführter massengelabelter Synthesen sowie massengelabelten Bausteinen L(randomisierte Position). In den randomisierten Positionen werden verschiedene Basen mit jeweils verschiedenen Substituenten am PNA-Rückgrat (als Massentags) verwendet. Die Masse des jeweiligen PNA-Moleküls ist eindeutig dessen Sequenz zugeordnet.

### Figur 8

Berechnetes MALDI-Massenspektrum einer PNA-Bibliothek.
Zwei verschiedene Festphasensynthesen (eine davon massengelabelt) mit je 32 verschiedenen Sequenzen ergeben 64 verschiedene Massenpeaks, von denen jeder zu einer spezifische Sequenz aus einer PNA-Bibliothek mit 3 variablen Positionen mit vier Basen (jeweils A, C, G und T eingesetzt) zugeordnet ist. Der Rechnung liegen die in Tabelle 1 aufgeführten Substituenten zugrunde. Für die Berechung wurde das Computerprogramm MASP (© Dr. Christoph Steinbeck) verwendet.

### Figur 9

Mögliche PNA-Synthesebausteine für die kombinatorische Boc-Festphasensynthese, wie sie auch für die Synthese massenunterscheidbarer PNA-Bibliotheken verwendet werden. Es sind die in Tabelle 1 aufgeführten massengelabelten Bausteine dargestellt.

### Figur 10

### Sequenzanalyse von Sonden durch spezifische Bindungsbrüche.

Die verschiedenen Sequenzen einer Bibliothek von DNA-Sonden können massenspektrometrisch auch durch spezifische Bindungbrüche an den randomisierten Positionen (N = A, G, C oder T) identifiziert werden. Bereits während der Festphasensynthese wird an diesen Positionen eine Phosphorothioatfunktion eingeführt, an der die DNA spezifisch gespalten werden kann (z.B. mit lodoethanol). Dies ist schematisch, ohne Berücksichtigung der genauen chemischen Beschaffenheit der Fragmente, dargestellt. Die Masse der Fragmente erlaubt, da die Sequenz zum großen Teil bekannt ist, die eindeutige Bestimmung der Gesamtsequenz, ohne daß eine vollständige Sequenzierung erfolgen müßte. Der Substituent R1 bezeichnet eine beliebige weitere DNA-Sequenz, R2 eine weitere beliebige Sequenz oder -H. R3 bezeichnet -OH (Phosphodiester) oder Alkyl- (Alkylphosphonat).

### Figur 11

"Charge tagged" PNA Sonden Gemisch CCXCAGCC (X= A, G, C, T) hybridisiert an eine Ziel-DNA immobilisiert auf Eupergit, CCCCAGCC ist die komplementäre Sequenz. Das untere Spektrum zeigt das Ausgangsgemisch, das obere Spektrum eine deutlich bevorzugte Hybridisierung der komplementären CCCCAGCC Sequenz.

### Figur 12

Zur auf dem MALDI-Probenträger immobilisierten Ziel-DNA komplementäre "charge tagged" CCTCAGCC, detektiert nach Aufbringen der 4-Hydroxy-α-cyanozimtsäure-Matrix ohne Bewegung des Probenträgers während der Messung.

Die Beispiele erläutern die Erfindung.

### Beispiel 1: Darstellung des gesamten erfindungsgemäßen Verfahrens

Ein MALDI Target wird mit einer Substanz beschichtet, die eine Immobilisierung eines chemisch modifizierten PCR Produktes erlaubt (z.B. eine NH₂ funktionalisierte Oberfläche). Ein Array von Ziel-DNAs wird auf diese Oberfläche übertragen und gebunden. Das Array wird mit einer Bibliothek von Sonden, die in beschriebener Weise hergestellt wurde, hybridisiert. Komplementäre Bestandteile der Sondenbibliothek binden an die entsprechenden Ziel-DNAs. Anschließend wird gründlich gewaschen, um nicht gebundene Sonden zu entfernen. Die Matrix wird aufgebracht und das MALDI Target ins Massenspektrometer übertragen. Jeder Punkt, wo sich eine Ziel-DNA befindet wird mit dem Laser angesteuert und eine Massenanalyse der Sonden, die sich an diese Ziel-DNA gebunden haben, durchgeführt.

### Beissiel 2: Analyse einer mit Charge Tags versehenen PNA-Bibliothek

Eine Bibliothek von PNAs wird in einer Synthese, wie sie hier beschrieben ist, mit Charge Tags versehen. Die Bibliothek von PNAs wird in 50 % Acetonitril gelöst und verdünnt. Die MALDI Matrix (in diesem Fall eine 1:1 Mischung von α-Cyano-4-methoxyzimtsäure und α-Cyano-4-hydroxyzimtsäuremethylester in Aceton) wird auf das MALDI Target gegeben. Das Lösungsmittel verdunstet unmittelbar. Danach wird 0.5 ml der CT-PNA Lösung auf auf die getrocknete Matrix gegeben. Nach dem Verdunsten dieses Lösungsmittels wird das MALDI Target ins Massenspektrometer überführt und die Sonde analysiert. Das Resultat ist in Figur 5 gezeigt.

### Beispiel 3: Analyse eines Arrays von Ziel-DNAs durch CT-PNAs

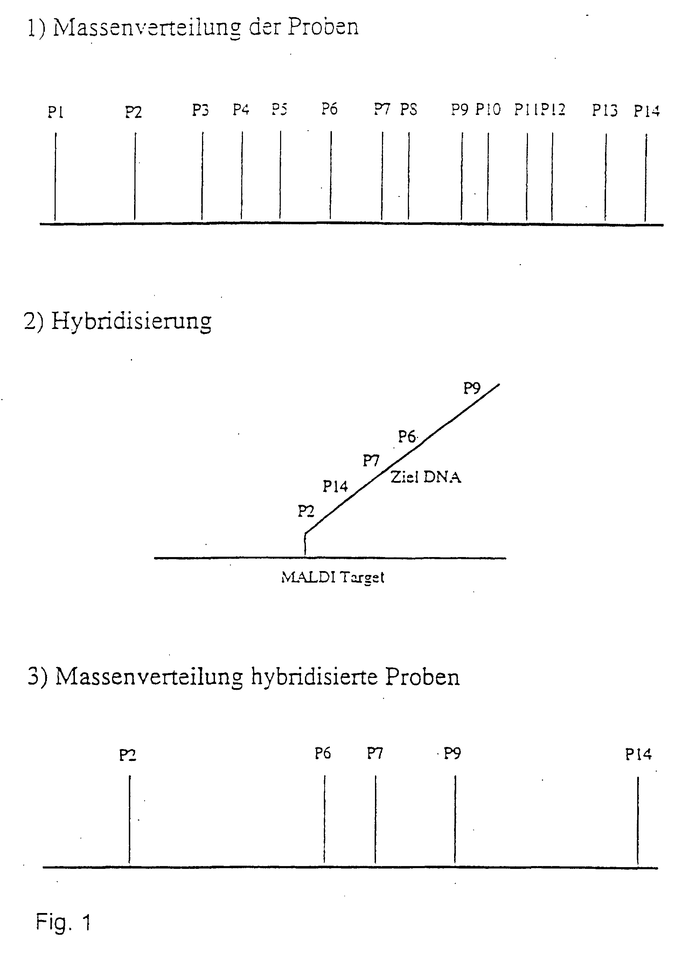

Eine Bibliothek von CT-PNAs, die auf die beschriebene Weise kombinatorisch hergestellt wurde, wird an ein Array von Ziel-DNAs, das auf einem MALDI Target immobilisiert ist, hybridisiert. Diejenigen Sonden, die eine komplementäre Sequenz in einer Ziel-DNA finden, werden gebunden. Danach wird das MALDI Target gewaschen, um alle übrigen Bestandteile der Bibliothek zu entfernen. Die Matrix wird auf das MALDI Target gegeben und das Target zur Analyse ins Massenspektrometer überführt (Figur 1). Eine unbekannte Ziel-DNA wird durch die hybridisierten Sonden charakterisiert. Ähnlichkeiten zwischen verschiedenen Ziel-DNAs zeichnen sich dadurch aus, daß die gleichen Sonden an sie binden.

### Beispiel 4: Analyse eines Arrays von Ziel-DNAs durch Sonden

Sonden werden individuell hergestellt und anschließend zu einer Bibliothek vereinigt. Wesentlich ist, daß keine Masse von zwei verschiedenen Sonden belegt wird. Diese Bibliothek von Sonden wird zur Hybridisierung an ein Array von immobilisierten Ziel-DNAs verwendet. Die nicht gebundenen Sonden werden abgewaschen. Die Matrix wird aufgetragen und die gebundenen Sonden im Massenspektrometer analysiert.

### Beispiel 5: Herstellung von Teilbibliotheken und MALDI-Analyse

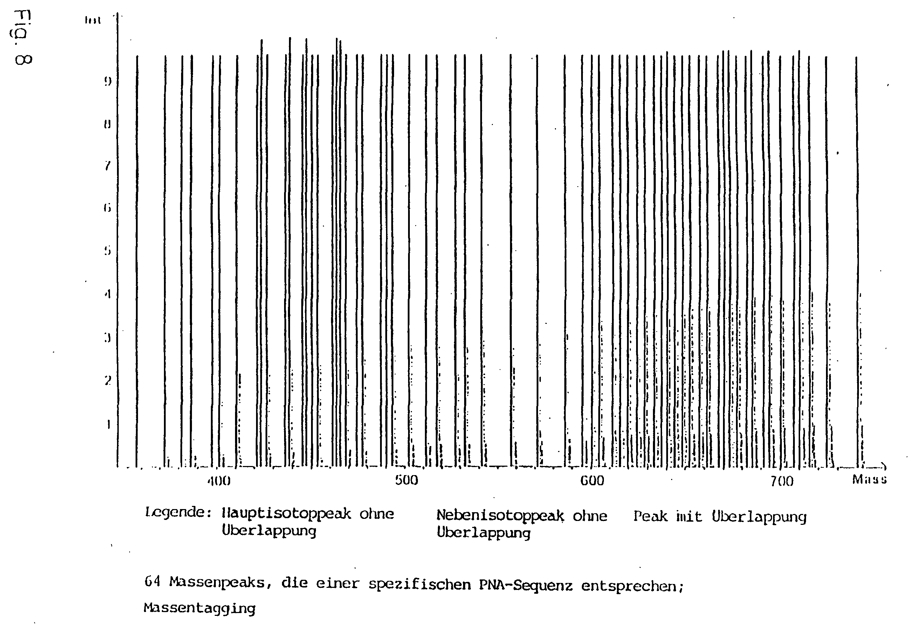

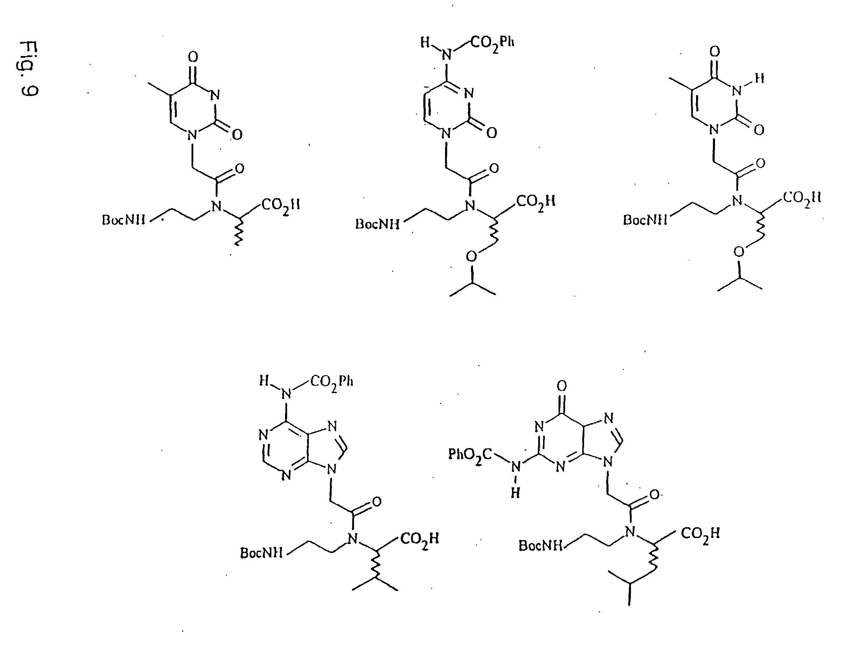

Ein bevorzugtes Beispiel für eine PNA-Bibliothek mit eindeutiger Beziehung der Masse zur Sequenz zeigen Figur 8 und Tabelle 1. In einer sonst beliebigen PNA sollen drei Positionen in der kombinatorischen Festphasensynthese variiert werden. Dies entspricht bei 4 verschiedenen Basen 64 möglichen Verbindungen. Im vorliegenden Beispiel werden zwei getrennte Synthesen mit den in Tabelle 1 gegebenen Bausteinen durchgeführt. Synthese 2 wird zusätzlich durch das Anbringen von zwei Valin-Einheiten massengelabelt. Jede Teilsynthese liefert 32 Verbindungen, die alle über ihre Masse unterscheidbar sind. Beide Teilbibliotheken können nun zur Bibliothek mit 64 verschiedenen Sequenzen mit jeweils spezifischer Masse vereinigt werden. Das berechnete MALDI-Massenspektrum für diese Bibliothek zeigt Figur 8. Die 64 Peaks überlappen nicht, jeder entspricht einer spezifischen Sequenz aus einer PNA-Bibliothek mit drei randomisierten Basenpositionen (je 4 Basen).

### Beispiel 6: Behandlung der Oberfläche des MALDI-Probenträgers

In einer bevorzugten Variante des Verfahrens wird die Oberfläche des MALDI-Probenträgers zunächst mit Silikat beschichtet und anschließend mittels Silanisierung Epoxy-funktionalisiert. Alternativ kann auch ein Epoxyfunktionalisiertes Acrylpolymer auf die Metalloberfläche aufgebracht werden. Die Ziel-DNA wird über die Epoxyfunktionen kovalent an die Oberfläche gebunden. In einer besonders bevorzugten Variante wird die zu immobilisierende DNA zuvor mit einer pimären Aminofunktion versehen. Die nicht umgesetzten. Expoxyfunktionen werden nachfolgend mit einem Überschuß eines Amins desaktiviert und das Verfahren nachfolgend entsprechend Beispiel 4 weitergeführt.

### Beispiel 7: Aufbau einer Sondenbibliothek mit 448 Sonden.

7 Synthesen werden nach der im letzten Beispiel beschriebenen Methode durchgeführt und anschließend die Massen/Charge tags gekoppelt.

| | Massenbereich |
|---|---|
| 1.64 Sonden: charge tag-TCP₁GAP₂GAP₃G | 2600-2800 Da |
| 2.64 Sonden: charge tag+200 Da mass tag- TCP₁AGP₂GAP₃G | 2800-3000 Da |
| 3.64 Sonden: charge tag+400 Da mass tag- TCP₁AGP₂AGP₃G | 3000-3200 Da |
| 4.64 Sonden: charge tag+600 Da mass tag- TCP₁AAP₂AGP₃G | 3200-3400 Da |
| 5.64 Sonden: charge tag+800 Da mass tag- TCP₁AAP₂GAP₃G | 3400-3600 Da |
| 6.64 Sonden: charge tag+1000 Da mass tag- TCP₁GAP₂GAP₃G | 3600-3800 Da |
| 7.64 Sonden: charge tag+1200 Da mass tag- TCP₁GAP₂AGP₃G | 3800-4000 Da |

In der vorgenannten Synthese-Serie stellt die sechste Synthese eine interne Kontrolle dar. Alternativ kann auch folgende Synthese durchgeführt werden: 6.64 Sonden: charge tag+ 1000 Da mass tag+ TCP₁GGP₂GAP₃G 3600-3800 Da.

### Beispiel 8: Kovalente Immobilisierung einer Ziel-DNA auf einer Metalloberfläche.

### 1. Beschichtung der Metalloberfläche mit Silikat und Silanen:

### Variante A:

50 mg Natriumtrisilikat (Aldrich) werden in 600 µL Wasser unter Erwärmen und Mischen gelöst und tropfenweise mit 100 µL Methanol versetzt. Die Lösung darf sich beim Stehenlassen innerhalb von 15 min nicht trüben. Auf die vorgereinigte Platte wird eine gleichmäßige Schicht dieser Lösung mit einem weichen Tuch aufgetragen und bei Raumtemperatur getrocknet. Die Platte wird 15 min bei 50°C weiter getrocknet und in Methanol gewaschen. Dann wird nochmals 15 min auf 50°C erwärmt und unpolymerisiertes Silikat mit Wasser abgewaschen. Die "verglaste" Metallplatte wird 20 min mit einer Lösung von 3-(Aminopropyl)-triethoxysilan (2% in Aceton:Wasser / 95:5) behandelt, mit Methanol gewaschen und bei 50°C getrocknet.

### Variante B: (bevorzugte Variante)

Der Silikatlösung wie unter A) beschrieben werden 5% 3-(Aminopropyl)-triethoxysilan zugesetzt und mit einem weichen Tuch eine gleichmaßige Schicht auf die Metallplatte aufgebracht. Nach dem Trocknen bei Raumtemperatur wird die Oberfläche für 30 s den Dämpfen konz. Salzsäure ausgesetzt, worauf sie sich milchig trübt. Die Platte wird 15 min bei 50°C getrocknet, mit Methanol und Wasser gewaschen und nachfolgend erneut getrocknet.

### Variante C:

Die vorgereinigte Metallplatte wird in einer Lösung von 3-(Aminopropyl)-triethoxysilan (2% in Aceton:Wasser / 95:5) 30 min silanisiert. Die Platte wird getrocknet, mit Methanol gewaschen, auf 50°C 15 min erwärmt und mit Wasser gewaschen.

### Variante D:

Analog zu den Varianten A, B und C ist die Funktionalisierung mit Thiolen durch Verwendung von 3-(Mercaptopropyl)-triethoxysilan möglich.

### 2. Vorbehandlung eines metallischen Trägers mit Epoxysilanen:

Ein metallischer Probenträger wird wie oben beschrieben mit Silikat beschichtet. Die Oberfläche wird mehrfach mit bidestilliertem Wasser gewaschen. Der getrocknete Probenträger wird mit einem 1:1-Gemisch aus 3-Glycidyloxypropyl-trimethoxysilan und Trimethoxy[2-(7-oxabicyclo[4.1.0]hept-3-yl)ethyl]silan (0.5µl/mm²) beschichtet und auf der Heizplatte 40 min. inkubiert. Die Plates werden gründlich mit Aceton gewaschen, 15 min. bei 55 °C getrocknet und um Vakuum aufbewahrt

### 3. Funktionalisierung von DNA mit einem Linker:

### Variante A:

Mit einer Phosphorothioat-Brücke synthetisierte DNA wird mit einem Überschuß SIAB (4-(lodacetamido)-benzoesäure N-hydroxysuccinimidylester, Pierce Chemical, Rockford, IL, USA) in DMF funktionalisiert. Das Lösemittel wird im Vakuum abgedampft, der gelbliche Rückstand mehrfach mit Essigsäureethylester gewaschen und anschließend getrocknet. Unter diesen Bedingungen reagiert ausschließlich die lodacetamido-Funktion. Der N-Hydroxy-succinimidester bleibt für die Weiterreaktion mit Aminofunktionen im Zuge der Immobilisierung erhalten.

### Variante B:

Das aminofunktionalisierte Oligonucleotid wird mit einem Überschuß SIAB in wasserfreiem DMSO bei Raumtemperatur umgesetzt. Die Aufreinigung erfolgt wie unter A). Unter diesen Bedingungen reagiert SIAB zu ca. 50% mit seiner lodoacetamido- und zu ca. 50% mit N-Hydroxy-succinimidester Funktion. Die Immobilisierung kann dann umgekehrt an einer entsprechend 1.D behandelten Metallplatte erfolgen.

### Variante C:

Die Varianten A) und B) können auch analog mit Halogenalkylcarbonsäure-NHS-Estern durchgeführt werden. Es sind jedoch längere Reaktionszeiten und erhöhte Temperaturen erforderlich.

### 4. Bindung von DNA an vorbehandelte Metallplatten:

### Variante A:

Die funktionalisierte DNA wird in einer gesättigten Lösung von Natriumacetat in wasserfreiem DMSO gelöst und auf die beschichtete Metallplatte aufgebracht. Nach 30 min Reaktionszeit wird das verbliebene Lösemittel entfernt und die Metallplatte zuerst mit 1 M Ammoniumchloridlösung und dann mit bidestilliertem Wasser derart gewaschen, daß eine gegenseitige Kontamination der benachbarten Spots vermieden wird. Dieser Vorgang wird dreimal wiederholt. Dann wird mit viel bidestilliertem Wasser nachgespült und die Metallplatte bis zur anschließenden Hybridisierung im Vakuum aufbewahrt.

### Variante B:

Analog zu A) ist auch die nicht kovalente Immobilisierung mit unmodifizierter DNA möglich, In diesem Fall wird im Waschprozeß auf die 1 M Ammoniumchloridlösung verzichtet.

### Variante C:

Die Immobilisierung von Ziel-DNA auf MALDI-Targets kann auch folgendermaßen durchgeführt werden. Die Amino-funktionalisierte DNA (1,5 nmol) wird in Immobilisierungspuffer (1M K₂HPO₄/KH₂PO₄ pH 7,5) aufgenommen. Die durch Silanisierung funktionalisierten Oberflächen werden mit dieser Lösung überschichtet und 16-20 h bei Raumtemperatur stehengelassen. Die Plates werden anschließend mehrfach mit dem Hybridisationspuffer gewaschen und getrocknet.

### Variante D:

Die Immobilisierung von Ziel-DNA an einer Epoxyfunktionalisierten Festphase kann wie folgt durchgeführt werden. 10 mg Support (Eupergit C250 L, Röhm Pharma Polymere) werden in 1 ml Immobilisierungspuffer (1M K₂HPO₄/KH₂PO₄ H 7,5) suspendiert. 1,5 nmol der zu immobilisierenden Ziel-DNA werden zugesetzt und 24 h bei Raumtemperature unter sanftem Schütteln inkubiert. Der Überstand wird abgenommen und die nicht umgesetzten Epoxyfunktionen durch Behandlung mit 1 M Glycinlösung bei Raumtermperatur 24 h desaktiviert. Der Überstand wird abgenommen, der Support mehrfach gewaschen. Die immobilisierte DNA kann so bei -20 °C über längere Zeit aufbewahrt werden.

### 5. Hybridisierung und Probenaufbereitung:

Die Hybridisierung an die immobilisierte Ziel-DNA mit PNA-Sonden (oder Nucleinsäure-Sonden) erfolgt in PBS-Puffer bei einer den jeweiligen Sonden angepaßten Temperatur, Das Target wird mehrfach zuerst mit PBS-Puffer, dann mit bidestilliertem Wasser nachgewaschen. Die Metallplatte wird im Vakuum getrocknet und anschließend mit der MALDI-Matrix (α-Cyano-4-hydroxyzimtsäure, 1% in Aceton; oder analog der entsprechende Methylester für das Verfahren mit "charge tagged" PNAs/Nucleinsäuren, oder als bevorzugte Variante ein 1:1 Gemisch des α-Cyano-4-hydroxyzimtsäuremethylesters mit α-Cyano-4-methoxyzimtsäure für die "charge tagged" PNAs) getrennt für jeden Spot beschichtet. Alternativ kann auch eine flächige Beschichtung mit einem Matrix-Spray durchgeführt werden. Durch den schnellen Trocknungsprozeß wird eine Diffusion innerhalb des Arrays vermieden.

### 5. Bevorzugte Variante

In der bevorzugten Variante erfolgt die Bindung einer mit einer Phosphorothioat-Brücke synthetisierten und mit SIAB nach 2.A funktionalisierten DNA an eine nach 1.B hergestellten Oberfläche auf einer Metallplatte durch Reaktion in wasserfreiem DMSO bei Raumtemperatur (30 min). Nach der Hybridisierung mit der charge getaggten Sonden-PNA/DNA wird das MALDI-Target mit bidestilliertem Wasser gewaschen, getrocknet und flächig getrennt für jeden Spot mit einem Gemisch aus α-Cyano-4-hydroxyzimtsäuremethylester und α-Cyano-4-methoxyzimtsäure (wie unter 4.) beschichtet.

### 6. Proteinbeschichtung:

Eine weitere Variante besteht darin die Metalloberfläche das MALDI Targets mit Protein zu beschichten. Gene32, ein Protein das sequenzunspezifisch einzelsträngige DNA bindet, ist dazu geeignet. Nach der Beschichtung des Targets mit diesem Protein kann ein Array von Ziel-DNAs daraufgelegt werden. Wenn das Array der Ziel-DNAs aus cDNA besteht, sind diese in der PCR mit oligo-dT (z.B. dTTTTTTTTTTTTT) geprimt worden. Oligo-dT wechselwirkt stark mit Gene32. Die kovalente Bindung des oligo-dT zum gene32 kann durch ein Photocrosslinking mit kurzem UV-Licht erreicht werden.^{36,37} Nach dieser Immobilisierung auf dem MALDI Target kann eine Bibliothek von Sonden in der beschriebenen Art zur Analyse des Arrays von Ziel-DNAs verwendet werden. Es bieten sich auch sequenzspezifische Protein/DNA-Wechselwirkungen an, wie z.B. GCN4/AP1.

Eine weitere Variante ist eine biotinylierte Ziel-DNA an ein mit Streptavidin beschichtetes magnetic bead zu binden. Diese Ziel-DNA wird mit einer Sondenbibliothek analysiert und anschließend die magnetischen Teilchen auf das MALDI Target übertragen, wo die Sonden durch die Matrix und leichtes Aufheizen auf die Matrix übertragen werden.

**Tabelle 1:**

| An den PNA-Untereinheiten angebrachte Substituenten zur Herstellung einer massengelabelten Bibliothek mit eindeutiger Massen/Sequenz Beziehung. | | | |
|---|---|---|---|
| Base | Position 1 | Position 2 | Position 3 |
| A | H | *i*Pr | H |
| T | H | Me | *i*PrOCH₂ |
| C | H | H | *i*PrOCH₂ * |
| G | H | *i*Bu | H* |

| | | | |
|---|---|---|---|
| * : Zweite Synthese, mit 2 Valin-Einheiten massengelabelt. Die entsprechenden Synthesebausteine zeigt Fig. 9. | | | |

### Literatur

¹ Hoheisel, J.D. and Lehrach, H. 1993. Use of reference libraries and hybridsationfingerprint for relational genome analysis. FEBS. 325: 118-122.
² Scholler, P., Karger, A.E., Meier-Ewert, S., Lehrach, H., Delius, H. and Hoheisel, J.D. 1995. Fine-mapping of shotgun template-libraries; an efficient strategy for the systematic sequencing of genomic DNA. Nucleic Acids Res. 23: 3842-3849.
³ Brenner, S. 1994. Methods for sorting polynucleotides using oligonucleotide tags. US Patent. 5,604,097.
⁴ Brenner, S. 1995. Minimally cross-hybridizing sets of oligonucleotide tags. US Patent. 5,635,400.
⁵ Guo, Z., Guilfoyle, R.A., Thiel, A.J., Wang, R. and Smith, L.M. 1994. Direct fluorescence analysis of genetic polymorphisms by hybridization with oligonucleotides arrays of glass supports. Nucleic Acids Res. 22: 5456-5465.
⁶ Church, G.M. and Kieffer-Higgins, S. 1988. Multiplex DNA sequencing. Science. 240: 185-188.
⁷ Church, G.M. and Kieffer-Higgins, S. 1990. Multiplex analysis of DNA. US Patent. 5,149,625.
⁸ Arlinghaus, H.F., Kwoka, M.N., Guo, X.-Q. and Jacobson, K.B. 1997. Multiplex DNA sequencing and diagnostics by hybridization with enriched stable isotope labeis. Anal. Chem. 69: 1510-1517.
⁹ Nielsen, P.E., Buchardt, O., Eghoim, M. and Berg, R.H. 1993. Peptide nucleic acids. US Patent. 5,539,082.
¹⁰ Buchardt, O., Egholm, M., Berg, R.H. and Nielsen, P.E. 1993. Peptide nucleic acids and their potential applications in biotechnology. Trends in Biotechnology, 11: 384-386.
¹¹ Karas, M. and Hillenkamp, F. 1988. Laser desorption ioniztion of proteins with molecular masses exceeding 10000 daltons. Anal. Chem. 60: 2299-2301.
¹² Vestal, M.L., Juhasz, P. and Martin, S.A. 1995. Delayed extraction matrix-assisted laser desorption/ionization time-of-flight mass spectrometry. Rapid Commun. Mass Spectrom. 9: 1044-1050.
¹³ Gut, I.G. and Beck, S. 1995. DNA and Matrix Assisted Laser Desorption lonization Mass Spectrometry. Molecutar Bioogy: Current Innovations and Future Trends. 1: 147-157.
¹⁴ Gut, I.G. and Beck, S. 1995. A procedure for selective DNA alkylation and detection by mass spectrometry. Nucleic Acids Res. 23: 1367-1373.
¹⁵ Gut, I.G. and Beck, S. 1995. Method of nucleic acid analysis. Patent WO96/27681.
¹⁶ Gut, I.G., Jeffery, W.A., Pappin, D.J.C. and Beck, S. 1997. Analysis of DNA by 'charge tagging' and matrix-assisted laser desorption/ionization mass spectrometry. Rapid Commun. Mass Spectrom. 11: 43-50.
¹⁷ Butler, J.M., Jiang-Baucom, P., Huang, M., Belgrader, P. and Girard, J. 1996. Peptide nucleic acid characterization by MALDI-TOF mass spectrometry. Anal. Chem. 68: 3283-3287.
¹⁸ Keough, T., Baker, T.R., Dobson, R.L.M., Lacey, M.P., Riley, T.A., Hasselfield, J.A. and Hesselberth, P.E. 1993. Antisense DNA oligonucleotides II: the use of matrix-assisted laser desorption/ionization mass spectrometry for the sequence verification of methylphosphonate oligodeoxyribonucleotides. Rapid Commun. Mass Spectrom. 7: 195-200.
¹⁹ Ross, P.L., Lee, K. and Belgrader, P. 1997. Discrimination of singlenucleotide polymorphisms in human DNA using peptide nucleic acid probes detected by MALDI-TOF mass spectrometry. Anal. Chem. 69: 4197-4202.
²⁰ Lowe, G. 1995. Combinatorial Chemistry. Chem. Soc. Rev. 24: 309.
²¹ Jung, G., Früchtel, J.S. 1996. Organische Chemie an fester Phase. Angew. Chem. 108: 19-46.
²² Choong, I.C., Ellman, J.A. 1996. Solid-Phase Synthesis: Applications to Combinatorial Libraries. Ann. Reports in Med. Chem. 31: 309.
²³ Pirrung, M.C. 1997. Spatially Adressable Combinatorial Libraries. Chem. Rev. 97: 473.
²⁴ Cook, P.D., Kiely, J. and Sprankle, K. 1994. Peptide nucleic acid combinatorial libraries and improved methods of synthesis. US Patent. 5,539,083.
²⁵ Nielsen, P.E. Peptide nucleic acids: a new dimension to peptide libraries and aptamers. Methods in Enzymology. 426-433.
²⁶ Metzger, J.W., Stevanovic, S., Brünjes, J., Wiesmüller, K.-H., Jung, G. 1994. Electrospray Mass Spectrometry amd Multiple Sequence Analysis of Synthetic Peptide Libraries Methods: A Companion to Methods in Enzymology 6: 425-431.
²⁷ Loo, J.A., DeJohn, D.E., Andrews, P.C. 1996. Application of Mass Spectrometry for Characterizing and Identifying Ligands from Combinatorial Libraries. Ann. Reports in Med. Chem. 31:319.
²⁸ Pomerantz, S.C., McCloskey, J.A., Eaton, B.C., 1997. Deconvolution of Combinatorial Oligonucleotide Libraries by Electrospray lonization Tandem Mass Spectrometry. J. Am. Chem. Soc. 119: 3861.
²⁹ Carr, S.A., Benkovic, S.J., Winograd, N. 1996. Evaluation of Mass Spectrometric Methods Applicable to the Direct Analysis of Non-Peptide Bead-Bound Combinatorial Libraries. Anal. Chem. 68: 237.
³⁰ Uhlen, M. et al. 1988, Nucleic Acids Res. 16, 3025-3038.
³¹ Chrisey, L.A., Lee, G.U. and O'Ferrall, C.E. 1996. Covalent attachment of synthetic DNA to self-assembled monolayer films. Nucleic Acids Res. 24: 3031-3039.
³² Timofeev, E.N., Kochetkova, S.V., Mirzabekov, A.D. und Florentiev, V.L. 1996. Regioselective immobilization of short oligonucleotides to acrylic copolymer gels. Nucleic Acids Res. 24: 3142-3148.
³³ O'Donnell, M.J., Tang, K., Köster, H., Smith, C.L. and Cantor, C.R. 1997. High-density, covalent attachment of DNA to silicone wafers for analysis by MALDI-TOF mass spectrometry. Anal. Chem. 69: 2438-2443.
³⁴ Cantor, C.R. 1995. Methods of preparing probe array by hybridization. US Patent. 5,631,134.
³⁵ Bruick, R.K., Koppitz, M., Joyce, G.F. and Orgel, L.E. 1997. A simple procedure for constructing 5'-amino-terminated oligodeoxynucleotides in aqueous solution. Nucleic Acids Res. 25: 1309-1310.
³⁶ Hockensmith, J.W., Kubasek, W.L., Vorachek, W.R. and von Hippel, P.H. 1993. Laser cross-linking of proteins to nucleic acids. J. Bio. Chem. 268: 15712-15720.
³⁷ von Hippel, P.H. 1994. Protein-DNA recognition: New perspectives and underying themes. Science. 263: 769-770.

## Patentansprüche

1. Verfahren zum Nachweis einer Nucleotidsequenz in einem Nucleinsäuremolekül, das die folgenden Schritte umfaßt:
(a) Hybridisierung von Nucleinsäuremolekülen mit einem Satz von Sonden unterschiedlicher Nucleobasensequenzen, wobei jede Sonde eine von allen anderen Sonden verschiedene Masse aufweist;
(b) Abtrennung der nicht hybridisierten Sonden;
(c) Kontaktierung der hybridisierten Sonden mit einer Matrix, welche die Desorption der Sonden durch einen Laserstrahl unterstützt;
(d) Analyse der hybridisierten und von der Matrix umgebenen Sonden auf einem aus elektrisch leitfähigem Material bestehenden Probenträger in einem Massenspektrometer; und
(e) Bestimmung der Nucleinsäuremoleküle, welche die Sequenz aufweisen,
wobei die Positionen der Sonden auf dem Probenträger eine Zuordnung zu dem damit hybridisierenden Nucleinsäuremolekül erlaubt.

2. Verfahren nach Anspruch 1, wobei die Nucleinsäuremoleküle vor oder nach Schritt (a) auf die Oberfläche eines Trägers übertragen werden.

3. Verfahren nach Anspruch 2, wobei die Oberfläche des Trägers die Oberfläche des aus elektrisch leitfähigem Material bestehenden Probenträgers ist.

4. Verfahren nach Anspruch 2, wobei vor Schritt (c) der Träger mit den an seiner Oberfläche aufgebrachten Nucleinsäuremolekülen, die die hybridisierten Sonden tragen, auf den aus leitfähigem Material bestehenden Probenträger aufgebracht wird.

5. Verfahren nach Anspruch 2, wobei die hybridisierten Sonden vor dem, nach dem oder durch den Kontakt mit der Matrix in Schritt (c) von den immobilisierten Nucleinsauremolekülen abgelöst und auf den aus leitfähigem Material bestehenden Probenträger aufgebracht werden.

6. Verfahren nach einem der Ansprüche 1 bis 5, wobei der Probenträger eine metallische, eine mit Glas beschichtete oder eine chemisch modifizierte Oberfläche aufweist.

7. Verfahren nach einem der Ansprüche 1 bis 6, wobei die Immobilisierung der Nucleinsäuremoleküle an der Probenträgeroberfiäche über eine NH₂-, Epoxy- oder SH-Funktion, vermittels der Beschichtung der Probenträgeroberfläche mit einem Silikat oder Silan, über eine Protein-Substrat-, Protein-Protein- oder eine Protein-Nucleinsäure-Interaktion, oder über eine Wechselwirkung zweier hydrophober Komponenten erfolgt.

8. Verfahren nach Anspruch 7, wobei die Protein-Substrat-Interaktion eine Biotin-Streptavidinbindung oder eine Antikörper-Antigenbindung ist.

9. Verfahren nach Anspruch 7, wobei die Protein-Nucleinsäureinteraktion eine Gene32-Nucleinsäurebindung ist.

10. Verfahren nach einem der Ansprüche 1 bis 9, wobei die Sonden Nucleinsäuren sind, die mit einem Massen-Tag versehen sind.

11. Verfahren nach Anspruch 10, wobei der Massen-Tag zugleich Ladungs-Tag ist.

12. Verfahren nach Anspruch 10, wobei die Nucleinsäuren zusätzlich mit einem Ladungs-Tag versehen sind.

13. Verfahren nach einem der Ansprüche 1 bis 12, wobei die Sonden modifizierte Nucleinsäuremoleküle sind.

14. Verfahren nach Anspruch 13, wobei die modifizierten Nucleinsäuremoleküle PNAs, alkylierte Phosphorothioatnucleinsäuren oder Alkylphosphonatnucleinsäuren sind.

15. Verfahren nach einem der Ansprüche 1 bis 14, wobei die Sonden durch kombinatorische Festphasen-Synthese hergestellt werden.

16. Verfahren nach Anspruch 15, wobei unterschiedliche Basenbausteine derart markiert sind, daß die aus ihnen synthetisierten Sonden jeweils über ihre Massen im Massenspektrometer unterscheidbar sind.

17. Verfahren nach Anspruch 16, wobei die Markierung eine Methyl-, Ethyl-, Propyl-, eine verzweigte oder unverzweigte Alkyl-, eine halogensubstituierte verzweigte oder unverzweigte Alkyl-, Alkoxyalkyl-, Alkylaryl-, Arylalkyl-, Alkoxyaryl- oder Aryloxyalkylgruppe ist, oder eine von deren deuterierten oder sonstigen isotopen Varianten.

18. Verfahren nach einem der Ansprüche 14 bis 17, wobei die Sonden mit mindestens einer Modifikation in einer definierten Position von randomisierten Nucleotiden entfernt versehen sind, die eine Spaltung der Sonde erlaubt.

19. Verfahren nach Anspruch 18, wobei die Modifikation die Einführung einer Phosphorothioatgruppe und/oder einer RNA-Base und/oder einer Phosphotriesterbindung in die Sonde ist.

20. Verfahren nach einem der Ansprüche 1 bis 19, wobei die Matrix eine Lösung aus α-Cyano-4-hydroxyzimtsäure in Aceton im Verhältnis 1:9 bis 9:1, vorzugsweise im Verhältnis 1:1, oder ein Gemisch aus α-Cyano-4-hydroxyzimtsäuremethylester und α-Cyano-4-methoxyzimtsäure oder Sinapinsäure oder deren Methylderivate im Verhältnis 1:9 bis 9:1, vorzugsweise im Verhältnis 1:1, ist.

21. Verfahren nach einem der Ansprüche 1 bis 19, wobei die Matrix α-Cyano-4-hydroxyzimtsäure oder ein Gemisch aus α-Cyano-4-hydroxyzimtsäuremethylester und α-Cyano-4-methoxyzimtsäure oder α-Cyano-4-hydroxyzimtsäure oder Sinapinsäure oder deren Methylderivate im Verhältnis 1:99 bis 99:1, vorzugsweise im Verhältnis 1:1 ist, die als Lösung in Aceton, Isopropanol, Acetonitril, Ethanol, Methanol oder Wasser oder in einem Gemisch aus zweien oder mehreren dieser Lösungsmittel auf den MALDI-Probenträger aufgebracht wird.

22. Verfahren nach einem der Ansprüche 1 bis 21, wobei die Sonden als Teilbibliotheken hergestellt werden, die mit unterschiedlichen Massenund/oder Ladungs-Tags versehen sind.

23. Kit enthaltend
(a) einen Satz von Sonden wie definiert in einem der Ansprüche 11 bis 18 und/oder
(b) einen Probenträger, der vorbehandelt ist und dadurch die Bindung eines Arrays von Ziel-DNAs erlaubt und/oder bereits gebundene Ziel-DNAs enthält.

## Claims

1. Method for the detection of a nucleotide sequence in a nucleic acid molecule comprising the following steps:
(a) hybridization of nucleic acid molecules with a set of different nucleotide base sequences, wherein each probe exhibits a mass different to the mass of all the other probes;
(b) separation of the non-hybridized probes;
(c) contact of the hybridized probes with a matrix supporting the desorption of the probes by means of a laser beam;
(d) analysis of the probes hybridized and surrounded by the matrix on a probe support consisting of electrically conductive material in a mass spectrometer; and
(e) determination of the nucleic acid molecules exhibiting the sequence, wherein the positions of the probes on the probe support allow for an allocation to the nucleic acid molecule hybridizing therewith.

2. Method according to claim 1, wherein the nucleic acid molecules are transferred to the surface of a carrier before or after step (a).

3. Method according to claim 2, wherein the surface of the carrier is the surface of the probe support consisting of conductive material.

4. Method according to claim 2, wherein before step (c) the carrier with the nucleic acid molecules which are applied to its surface and which carry the hybridized probes is applied to the probe support consisting of conductive material.

5. Method according to claim 2, wherein in step (c) the hybridized probes are separated from the immobilized nucleic acid molecules before, after or through the contact with the matrix.

6. Method according to any one of claims 1 to 5, wherein the probe support has a surface which is metal, coated with glass or chemically modified.

7. Method according to any one of claims 1 to 6, wherein the immobilization of the nucleic acid molecules on the probe support is carried out through a NH₂, an epoxy- or a SH-function by means of coating of the probe support surface with silicate or silane, via protein-substrate-, protein-protein- or a protein-nucleic acid-interaction or via interaction between two hydrophobe components.

8. Method according to claim 7, wherein the protein-substrate-interaction is a biotinstreptavidin-bond or an antibody-antigen-bond.

9. Method according to claim 7, wherein the protein-nucleic acid-interaction is a Gene32-nucleic-acids-linking.

10. Method according to any one of claims 1 to 9, wherein the probes are nucleic acids with a mass tag.

11. Method according to claim 10, wherein the mass tag is also a charge tag.

12. Method according to claim 10, wherein the nucleic acids have an additional charge tag.

13. Method according to any one of claims 1 to 12, wherein the probes are modified nucleic acid molecules.

14. Method according to claim 13, wherein the modified nucleic acid molecules are PNAs, alkylated phosphorothioate nucleic acids or alkylphosphonate nucleic acids.

15. Method according to any one of claim 1 to 14, wherein the probes are produced by combinatorial solid phase synthesis.

16. Method according to claim 15, wherein various base building blocks are marked in such a way that each probe synthesized from them can be differentiated from other probes via its mass in the mass spectrometer.

17. Method according to claim 16, wherein the marking consists of a methyl-, ethyl-, propyl-, a branched or non-branched alkyl-, a halogen-substituted branched or unbranched alkyl-, alkoxyalkyl-, alkylaryl-, arylakyl-, alkoxyaryl- or aryloxyalkyl-group, or one of its deuterated or otherwise isotopic variants.

18. Method according to any one of claims 14 to 17, wherein the probes have at least one modification in a defined position away from randomized nucleotides which allows for cleavage of the probe.

19. Method according to claim 18, wherein the modification is the introduction of a phosphorothioate group and/or an RNA base and/or a phosphotriester bond in the probe.

20. Method according to any one of claims 1 to 19, wherein the matrix is a solution of α-cyano-4-hydroxy cinnamic acid in acetone at a ratio of 1:9 to 9:1, preferably at a ratio of 1:1, or a mixture of α-cyano-4-hydroxy cinnamic acid methyl ester and α-cyano-4-methoxy cinnamic acid or sinapic acid or its methyl derivative at a ratio of 1:9 to 9:1, preferably at a ratio of 1:1.

21. Method according to any one of claim 1 to 19, wherein the matrix is a solution of α-cyano-4-hydroxy cinnamic acid in acetone at a ratio of 1:9 to 9:1, preferably at a ratio of 1:1, or a mixture of α-cyano-4-hydroxy cinnamic acid methyl ester and α-cyano-4-methoxy cinnamic acid or sinapic acid or its methyl derivative at a ratio of 1:9 to 9:1, preferably at a ratio of 1:1, which is applied as solution in acetone, isopropanol, acetonitril, ethanol, methanol or water or in a mixture of two or more of those solvents to the MALDI probe support.

22. Method according to any one of claims 1 to 21, wherein the probes are produced as partial libraries having different mass and/or charge tags.

23. Kit containing
(a) a set of probes as defined in any one of claims 11 to 18 and/or
(b) a probe support which has been pre-treated and, thus, allows for the linking of an array of target DNAs and/or contains already bound target DNAs.

## Revendications

1. Pracédé pour détecter une séquence nucléotidique dans une molécule d'acide nucléique, qul comprend les étapes suivantes:
(a) hybridation de molécules d'acide nucléique avec un ensemble de sondes ayant des séquences de nucléobases différentes, chaque sonde ayant une masse différente de l'ensemble des autres sondes ;
(b) séparation des sondes non-hybridées ;
(c) mise en contact des sondes hybridées avec une matrice qui facilite la désorption des sondes à l'aide d'un faisceau laser ;
(d) analyse des sondes hybridées et entourées de la matrice, sur un porte-échantillon, constitué d'un matériau conducteur de l'électricité, dans un spectrométre de masse ; et
(e) détermination des molécules d'acide nucléique qui comportent la séquence,
les positions des sondes sur le porte-échantillon permettant une affectation à la molécule d'acide nucléique qui doit y être hybridée.

2. Procédé selon la revendication 1, dans lequel les molécules d'acide nucléique sont, avant ou après l'étape (a), transférées sur la surface d'un support.

3. Procédé selon la revendication 2, dans lequel la surface du support est la surface du porte-échantillon constitué d'un matériau conducteur de l'électricité.

4. Procédé selon la revendication 2, dans lequel, avant l'étape (c), le support comportant les molécules d'acide nucléique appliquées sur sa surface, et qui portent les sondes hybridées, est appliqué sur le porte-échantillon constitué du matériau conducteur.

5. Procédé selon la revendication 2, dans lequel les sondes hybridées sont, avant ou après contact, ou sous l'effet du contact avec la matrice dans l'étape (c), détachées des molécules d'acide nucléique immobilisées et sont appliquées sur le porte-échantillon constitué du matériau conducteur.

6. Procédé selon l'une des revendications 1 à 5, dans lequel le porte-échantillon comporte une surface métallique, une surface enduite de verre, ou une surface chimiquement modifiée.

7. Procédé selon l'une des revendications 1 à 6, dans lequel l'immobilisation des molécules d'acide nucléique sur la surface du porte-échantillon a lieu par l'intermédiaire dune fonction NH₂, époxy ou SH, par application d'un silicate ou d'un silane sur la surface du porte-échantillon, par l'intermédiaire d'une inter-action protéine-substrat, protéine-protéine ou protéine-acide nucléique, ou par une interaction de deux composants hydrophobes.

8. Procédé selon la revendicadon 7, dans lequel l'interaction protéine-substrat est une liaison biotine-streptavidine ou une liaison anticorps-antigène.

9. Procédé selon la revendication 7, dans lequel l'interaction protéine-acide nucléique est une liaison gène 32-acide nucléique.

10. Procédé selon l'une des revendications 1 à 9, dans lequel les sondes sont des acides nucléiques qui sont pourvues d'un tag de masse.

11. Procédé selon la revendication 10, dans lequel le tag de masse est aussi un tag de charge.

12. Procédé selon la revendication 10, dans lequel les acides nucléiques sont en outre pourvus d'un tag de charge.

13. Procédé selon l'une des revendications 1 à 12, dans lequel les sondes sont des molécules d'acide nucléique modifiées.

14. Procédé selon la revendication 13, dans lequel les molécules d'acide nucléique modifiées sont des PNA, des acides phosphorothloate-nuclélques alkylés ou des acides alkylphosphonate-nucléiques.

15. Procédé selon l'une des revendications 1 à 14, dans lequel les sondes sont préparées par une synthèse combinatoire en phase solide.

16. Procédé selon la revendication 15, dans lequel des composants de base différents sont marqués de façon que chacune des sondes synthétisées à partir d'eux puis-sent se distinguer par leur masse dans le spectromètre de masse.

17. Procédé selon la revendication 16, dans lequel le marquage est un groupe méthyle, éthyle, propyle, un groupe alkyle à chaîne droite ou ramifiée, un groupe alkyle, alcoxyalkyle, alkylaryle, arylalkyle, alcoxyaryle ou aryloxyalkyle halogéné à chaîne droite ou ramifiée, ou encore l'une de leurs variantes deutériées ou autrement isotopes.

18. Procédé selon l'une des revendications 14 à 17, dans lequel les sondes sont pourvues d'au moins une modification sur une position définie éloignée de nucléotides randomisés, qui permet une dissociation de la sonde.

19. Procédé selon la revendication 18, dans lequel la modification réside dans l'introduction d'un groupe phosphorothioate et/ou dune base d'ARN et/ou dune liaison phosphotriester dans la sonde.

20. Procédé selon l'une des revendications 1 à 19, dans lequel la matrice est une solution d'acide α-cyano-4-hydroxycinnamique dans l'acétone selon un rapport de 1:9 à 9:1, de préférence selon un rapport de 1:1, ou encore un mélange de l'ester méthylique de l'acide α-cyano-4-hydroxycinnamique et d'acide α-cyano-4-méthoxy-cinnamique ou d'acide sinapique, ou de ses dérivés méthylés, selon un rapport de 1:9 à 9:1 et de préférence selon un rapport de 1 :1.

21. Procédé selon l'une des revendications 1 à 19, dans lequel la matrice est l'acide α-cyano-4-hydroxy-cinnamique dans l'acétone selon un rapport de 1 :9 à 9 :1, de préférence selon un rapport de 1:1 ou un mélange de l'ester méthylique de l'acide α-cyano-4-hydroxycinnamique et d'acide α-cyano-4-méthoxy-cinnamique ou d'acide sinapique, ou de ses dérivés méthylés, selon un rapport de 1 :9 à 9 :1 et de préférence selon un rapport de 1 :1, qui est appliqué sur le port-échantillon pour MALDI sous forme d'une solution dans l'acétone, l'isopropanol, l'acétonitrile, l'éthanol, le méthanol ou l'eau, ou dans un mélange d'au moins deux de ces solvants.

22. Procédé selon l'une des revendications 1 à 21, dans lequel les sondes sont préparées à partir de banques partielles qui sont pourvues de différents tags de masse et/ou de charge.

23. Trousse contenant :
(a) un ensemble de sondes telles que défini dans l'une des revendications 11 à 18, et/ou
(b) un porte-échantillon, qui a subi un traitement préalable et de ce fait autorise la liaison dune matrice d'ADN-cibles et/ou contient des ADN-cibles déjà liés.
